(19) Europäisches Patentamt
European Patent Office
Office européen des brevets

(11) Veröffentlichungsnummer: **0 109 575**
**A2**

(12) # EUROPÄISCHE PATENTANMELDUNG

(21) Anmeldenummer: **83110605.9**

(22) Anmeldetag: **24.10.83**

(51) Int. Cl.³: **C 07 C 161/00**
C 07 D 213/64, C 07 D 333/46
A 01 N 41/02, A 01 N 43/40
A 01 N 43/10

(30) Priorität: **04.11.82 CH 6418/82**
**06.09.83 CH 4873/83**

(43) Veröffentlichungstag der Anmeldung:
**30.05.84 Patentblatt 84/22**

(84) Benannte Vertragsstaaten:
**BE CH DE FR GB IT LI NL**

(71) Anmelder: **F. HOFFMANN-LA ROCHE & CO. Aktiengesellschaft**

**CH-4002 Basel(CH)**

(72) Erfinder: **Frater, Georg, Dr.**
**Am Pfisterhölzli 22**
**CH-8606 Greifensee(CH)**

(72) Erfinder: **Suchy, Milos, Dr.**
**Grossplatzstrasse 3**
**CH-8122 Pfaffhausen(CH)**

(72) Erfinder: **Wenger, Jean, Dr.**
**Brunnenwiesenstrasse 1**
**CH-8610 Uster(CH)**

(72) Erfinder: **Winternitz, Paul, Dr.**
**Am Pfisterhölzli 50**
**CH-8606 Greifensee(CH)**

(74) Vertreter: **Lederer, Franz, Dr. et al,**
**Patentanwälte Dr. Lederer Franz Meyer-Roxlau Reiner F.**
**Lucile-Grahn-Strasse 22**
**D-8000 München 80(DE)**

(54) **Benzoesäurederivate, Verfahren und Mittel zu deren Herstellung.**

(57) Benzoesäurederivate der allgemeinen Formel

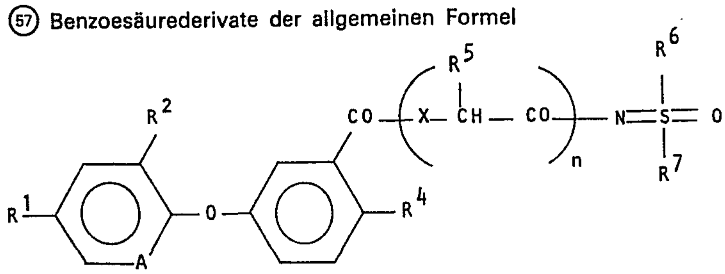

I,

worin A N oder $CR^3$,
$R^1$ Halogen oder Trifluormethyl,
$R^2$ und $R^3$, unabhängig voneinander, Wasserstoff oder Halogen,
$R^4$ Halogen, Nitro oder Cyano,
$R^5$ Wasserstoff oder Methyl,
$R^6$ -Alkyl und $R^7$ -Alkyl oder gegebenenfalls mit Halogen, -Alkyl, -Alkoxy und/oder Nitro substituiertes Aryl, oder $R^6$ und $R^7$ zusammen Tetra-, Penta- oder Hexamethylen,
n 0 oder 1,
X Sauerstoff oder $-NR^8-$
und $R^8$ Wasserstoff oder -Alkyl bedeuten.

Verfahren zu deren Herstellung, Unkrautbekämpfungsmittel, die diese Verbindungen als Wirkstoffe enthalten, sowie die Verwendung solcher Verbindungen bzw. Mittel zur Bekämpfung von Unkräutern. Zudem betrifft sie zur Herstellung dieser Verbindungen dienende Ausgangsmaterialien.

EP 0 109 575 A2

F.Hoffmann-La Roche & Co.Aktiengesellschaft, Basel/Schweiz



24. Okt. 1983

RAN 6102/33

# Benzoesäurederivate, Verfahren und Mittel zu deren Herstellung

Die Erfindung betrifft Benzoesäurederivate der allgemeinen Formel

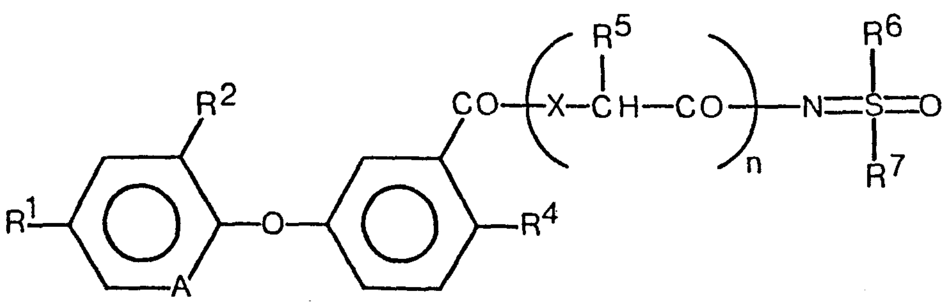

I

worin A N oder $CR^3$,

$R^1$ Halogen oder Trifluormethyl,

$R^2$ und $R^3$, unabhängig voneinander, Wasserstoff oder Halogen,

$R^4$ Halogen, Nitro oder Cyano,

$R^5$ Wasserstoff oder Methyl,

$R^6$ $C_{1-6}$-Alkyl und $R^7$ $C_{1-6}$-Alkyl oder gegebenenfalls mit Halogen, $C_{1-4}$-Alkyl, $C_{1-4}$-Alkoxy und/oder Nitro substituiertes Aryl, oder $R^6$ und $R^7$ zusammen Tetra-, Penta- oder Hexamethylen,

n 0 oder 1,

X Sauerstoff oder $-NR^8-$

und $R^8$ Wasserstoff oder $C_{1-6}$-Alkyl bedeuten.

Pa/24.8.83

Die Verbindungen der Formel I besitzen herbizide Eigenschaften und eignen sich somit als Unkrautbekämpfungsmittel oder als Wirkstoffe von Unkrautbekämpfungsmitteln. Somit umfasst die Erfindung auch Unkrautbekämpfungsmittel, die mindestens eine Verbindung der Formel I als Wirkstoff enthalten, Verfahren zur Herstellung dieser Verbindungen sowie die Verwendung solcher Verbindungen bzw. Mittel zur Bekämpfung von Unkräutern.

In der obigen Formel I umfasst der Ausdruck "Halogen" Fluor, Chlor, Brom und Jod, wobei Chlor bevorzugt ist, und der Ausdruck "Aryl" insbesondere Phenyl. Unter "$C_{1-6}$-Alkyl" bzw. "$C_{1-4}$-Alkyl" sind sowohl geradkettige als auch verzweigte Alkylgruppen zu verstehen, wie Methyl, Aethyl, n-Propyl, Isopropyl, n-Butyl, Neopentyl und n-Hexyl. Dies gilt auch für die $C_{1-4}$-Alkylgruppe enthaltende $C_{1-4}$-Alkoxygruppe. Wenn im Arylrest zwei oder mehr Substituenten vorhanden sind, können diese gleich oder verschieden sein.

Falls im Molekül asymmetrische Kohlenstoffatome vorhanden sind, treten die Verbindungen der Formel I in optisch aktiver Form auf. Auch durch das Vorliegen eines chiralen Schwefelatoms, d.h. für jene Verbindungen der Formel I, worin $R^6$ und $R^7$ unterschiedliche Bedeutung haben, tritt optische Isomerie auf. Die Formel I soll demnach die Racemate sowie all diese möglichen isomeren Formen umfassen.

Unabhängig voneinander bedeuten $R^1$ vorzugsweise Trifluormethyl, $R^2$ vorzugsweise Chlor und das allfällig vorhandene Symbol $R^3$ vorzugsweise Wasserstoff. Am bevorzugtesten sind gleichzeitig $R^1$ Trifluormethyl, $R^2$ Chlor und $R^3$ Wasserstoff.

$R^4$ bedeutet vorzugsweise Nitro.

Die bevorzugten Alkylreste von $R^6$ und $R^7$ sind diejenigen mit 1-3 Kohlenstoffatomen, insbesondere Methyl.

Eine besonders bevorzugte Verbindung der Formel I ist das N-[5-(o-Chlor-p-trifluormethyl-phenoxy)-2-nitrobenzoyl]-S,S-dimethyl-sulfoximin.

Das erfindungsgemässe Verfahren zur Herstellung der Verbindungen der Formel I ist dadurch gekennzeichnet, dass man

a) eine Säure der allgemeinen Formel

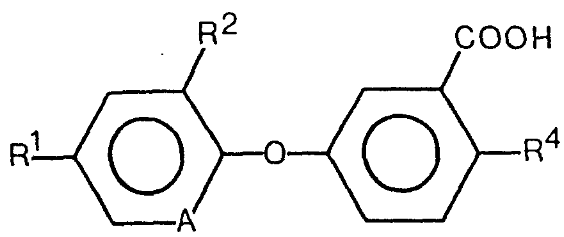

II

worin A, $R^1$, $R^2$ und $R^4$ die oben angegebenen Bedeutungen besitzen,

in Form eines reaktionsfähigen Derivats mit einem Sulfoximin der allgemeinen Formel

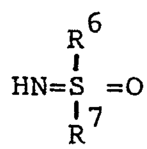

III

worin $R^6$ und $R^7$ die oben angegebenen Bedeutungen besitzen,

oder mit einem Alkalimetallsalz davon umsetzt,

b) ein Salz oder reaktionsfähiges Derivat einer Säure der oben angegebenen allgemeinen Formel II mit einer Verbindung der allgemeinen Formel

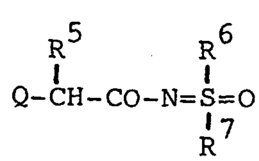

IV

worin $R^5$, $R^6$ und $R^7$ die oben angegebenen Bedeutungen besitzen und Q eine Abgangsgruppe bzw. Hydroxy bedeutet,

umsetzt,

c) eine Verbindung der allgemeinen Formel

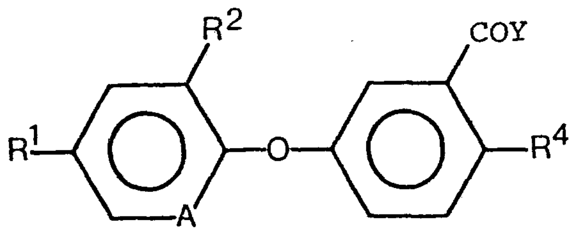

V

worin A, $R^1$, $R^2$ und $R^4$ die oben angegebenen Bedeutungen besitzen,

Y -NHR$^8$ oder Halogen bedeutet

und $R^8$ die oben angegebene Bedeutung besitzt,

mit einer Verbindung der allgemeinen Formel

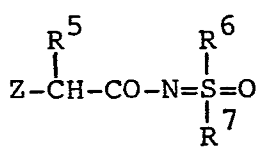

VI

worin $R^5$, $R^6$ und $R^7$ die oben angegebenen Bedeutungen besitzen

und Z im Falle von Y = -NHR$^8$ für eine Abgangsgruppe, wie Chlor, Brom, Jod, Mesyloxy oder Tosyloxy, und im Falle von Y = Halogen, wie Chlor oder Brom, für -NHR$^8$ steht,

umsetzt,

d) ein Phenol der allgemeinen Formel

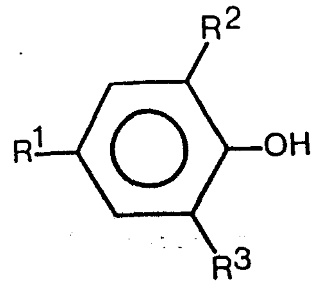

VII

worin $R^1$, $R^2$ und $R^3$ die oben angegebenen Bedeutungen besitzen,

oder ein Alkalimetallsalz davon mit einem Halogenid der allgemeinen Formel

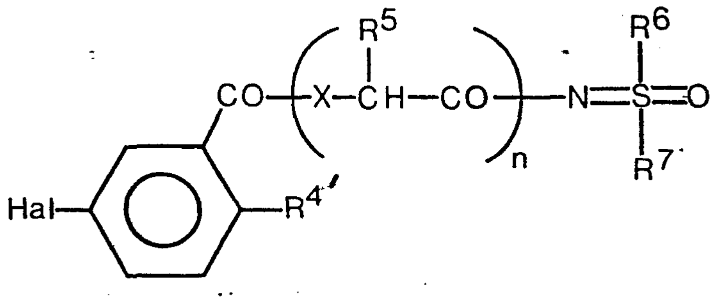

VIII

worin $R^5$, $R^6$, $R^7$, n und X die oben angegebenen Bedeutungen besitzen,

$R^{4'}$ Nitro oder Cyano bedeutet

und Hal Halogen, insbesondere Fluor, bedeutet,

umsetzt,

e) ein Hydroxybenzoesäurederivat der allgemeinen Formel

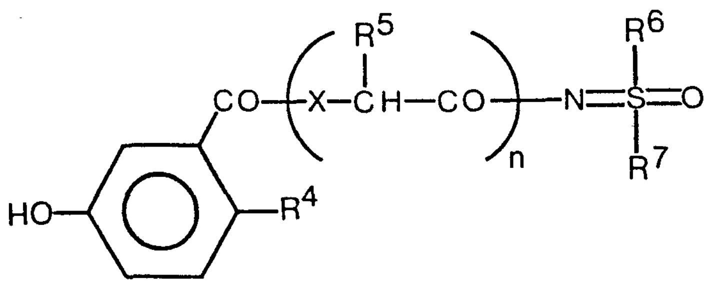

IX

worin $R^4$, $R^5$, $R^6$, $R^7$, n und X die oben angegebenen Bedeutungen besitzen,

oder ein Alkalimetallsalz davon mit einem Halogenid der allgemeinen Formel

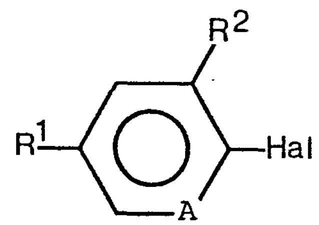 X

worin A, $R^1$ und $R^2$ die oben angegebenen Bedeutungen besitzen und Hal Halogen bedeutet, wobei, falls A $CR^3$ und $R^2$ und/oder $R^3$ Halogen darstellen, $R^1$ Trifluormethyl bedeutet,

umsetzt, oder

f) eine Verbindung der allgemeinen Formel

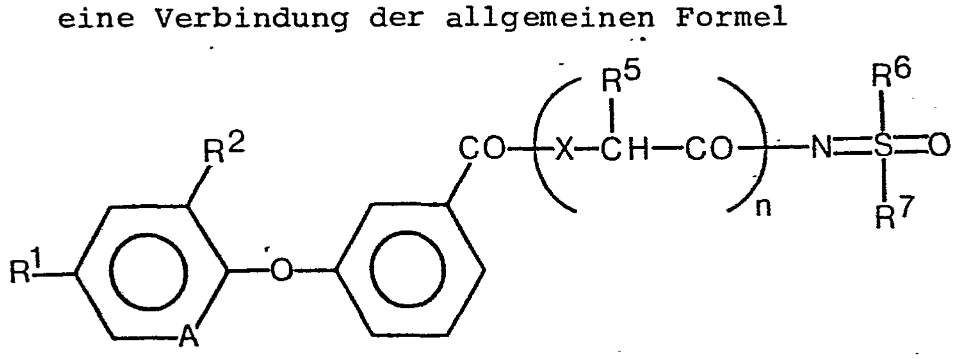 XI

worin A, $R^1$, $R^2$, $R^5$, $R^6$, $R^7$, n und X die oben angegebenen Bedeutungen besitzen,

nitriert.

Die Verfahrensvariante a) führt zu denjenigen Verbindungen der Formel I, worin n 0 bedeutet. Sie kann unter an sich bekannten Reaktionsbedingungen durchgeführt werden. Je nach Natur der Reaktionspartner kommen die folgenden Ausführungsformen in Frage:

Variante $a_1$): Man setzt ein reaktionsfähiges Derivat einer Säure der Formel II, wie den entsprechenden O-Acyl-1,3-dicyclohexylisoharnstoff, das entsprechende N-Acylimidazol oder Säureanhydrid, oder ein gemischtes Säureanhydrid, z.B. das aus der Säure II und Chlorameisensäureäthylester gebildete Säureanhydrid, mit einem Sulfoximin der Formel III um.

Variante $a_2$): Man setzt ein reaktionsfähiges Derivat einer Säure der Formel II, insbesondere ein Säurehalogenid, z.B. das Säurechlorid, mit einem Sulfoximin der Formel III in Gegenwart einer organischen Base, wie eines tertiären Amins, z.B. Triäthylamin, Dimethylanilin oder Pyridin, 1,5-Diaza-bicyclo[4,3,0]non-5-en, 1,8-Diaza-bicyclo[4,5,0]undec-7-en oder 1,4-Diaza-bicyclo[2,2,2]-octan, um.

Variante $a_3$): Man setzt ein reaktionsfähiges Derivat einer Säure der Formel II, insbesondere ein Säurehalogenid, z.B. das Säurechlorid, mit einem Alkalimetallsalz eines Sulfoximins der Formel III, wie dem Lithium-, Natrium- oder, vorzugsweise, Kaliumsalz, um.

In gewissen Fällen wird das reaktionsfähige Derivat der Säure der Formel II zweckmässigerweise in situ hergestellt und dies ohne Isolierung mit dem Sulfoximin der Formel III im gleichen Reaktionsmedium umgesetzt. So wird beispielsweise eine Säure der Formel II mit Dicyclohexylcarbodiimid zum entsprechenden O-Acyl-1,3-dicyclohexylisoharnstoff oder Säureanhydrid umgesetzt, und der Harnstoff bzw. das Anhydrid anschliessend im gleichen Reaktionsmedium mit dem Sulfoximin reagieren gelassen.

Die Umsetzung gemäss Verfahrensvariante a) erfolgt zweckmässigerweise in einem inerten organischen Verdünnungsmittel, wie einem aliphatischen oder cyclischen Aether, z.B. Diäthyläther, Tetrahydrofuran oder Dioxan, einem Kohlenwasserstoff, z.B. Toluol, oder einem halogenierten Kohlenwasserstoff, z.B. Methylenchlorid, und bei Temperaturen zwischen -20°C und 100°C, vorzugsweise zwischen 0°C und 70°C.

Die Verfahrensvariante b) führt zu denjenigen Verbindungen der Formel I, worin n 1 und X Sauerstoff bedeuten. Es handelt sich dabei um eine Veresterung, die nach an sich bekannten Methoden durchgeführt werden kann. So wird beispielsweise ein Salz einer Säure der Formel II

mit einer Verbindung der Formel IV, in der Q für eine Abgangsgruppe, insbesondere für Chlor, Brom, Jod, Mesyloxy oder Tosyloxy, steht, zweckmässigerweise in einem inerten Verdünnungsmittel und bei Temperaturen von etwa -20°C bis 150°C, vorzugsweise im Temperaturbereich von 0°C bis 100°C, umgesetzt. Es kommen als Salze der Säure der Formel II insbesondere Alkalimetallsalze, z.B. das Natrium-, Kalium- oder Lithiumsalz, Erdalkalimetallsalze, z.B. das Magnesium-, Calcium- oder Bariumsalz, Salze mit organischen Basen, wie Trialkylamine, z.B. Triäthylamin, 1,5-Diaza-bicyclo[4,3,0]non-5-en, 1,8-Diaza-bicyclo[4,5,0]-undec-7-en und 1,4-Diaza-bicyclo[2,2,2]octan, in Frage, wobei die Alkalimetallsalze bevorzugt sind. Die verwendbaren Verdünnungsmittel sind vorzugsweise inerte organische Lösungsmittel, wie aliphatische und cyclische Aether, z.B. Diäthyläther, Tetrahydrofuran und Dioxan, Aromaten, z.B. Benzol und Toluol, Dimethylformamid, Dimethylsulfoxid und Hexamethylphosphorsäuretriamid.

Das Salz der Säure der Formel II wird zweckmässigerweise in situ hergestellt, indem man die Säure mit einer geeigneten anorganischen Base, z.B. einem Alkalimetall- oder Erdalkalimetallcarbonat oder -bicarbonat, bzw. organischen Base, zum Salz umsetzt und dies anschliessend im gleichen Reaktionsmedium mit der Verbindung der Formel IV reagieren lässt.

Im Falle der Verwendung eines reaktionsfähigen Derivats der Säure der Formel II steht Q in der Formel IV für Hydroxy. Das reaktionsfähige Derivat der Säure der Formel II ist vorzugsweise ein Säurehalogenid, insbesondere das Säurechlorid, kann aber auch z.B. der entsprechende O-Acyl-1,3-dicyclohexylisoharnstoff oder das entsprechende N-Acylimidazol oder Säureanhydrid sein.

In gewissen Fällen wird das reaktionsfähige Derivat der Säure der Formel II zweckmässigerweise in situ hergestellt und dies ohne Isolierung mit der Verbindung der

Formel IV im gleichen Reaktionsmedium umgesetzt. So wird beispielsweise eine Säure der Formel II mit Dicyclohexylcarbodiimid zum entsprechenden O-Acyl-1,3-dicyclohexylisoharnstoff oder Säureanhydrid umgesetzt und dies anschliessend im gleichen Reaktionsmedium mit der Verbindung der Formel IV reagieren gelassen.

Die Umsetzung wird zweckmässigerweise in einem inerten Lösungsmittel, wie einem aliphatischen oder cyclischen Aether, z.B. Diäthyläther, Tetrahydrofuran oder Dioxan, einem aliphatischen oder aromatischen Kohlenwasserstoff, z.B. n-Hexan, Benzol oder Toluol, oder einem halogenierten, insbesondere chlorierten, Kohlenwasserstoff, z.B. Methylenchlorid oder Chloroform, und bei Temperaturen von etwa -20°C bis 150°C, vorzugsweise von 0°C bis 50°C, durchgeführt.

Bei Verwendung eines Säurehalogenides der Säure der Formel II wird die Umsetzung zweckmässigerweise in Gegenwart eines säurebindenden Mittels durchgeführt. Als geeignet erweisen sich anorganische Basen, z.B. Alkalimetall- und Erdalkalimetallcarbonate und -bicarbonate, sowie organische Basen, z.B. tertiäre Amine, insbesondere Triäthylamin und Pyridin, 1,5-Diaza-bicyclo[4,3,0]non-5-en, 1,8-Diaza-bicyclo[4,5,0]undec-7-en und 1,4-Diaza-bicyclo[2,2,2]octan.

Die Verfahrensvariante c) führt zu denjenigen Verbindungen der Formel I, worin n 1 und X $-NR^8-$ bedeuten.

Die Umsetzung einer Verbindung der Formel V, worin Y $-NHR^8$ bedeutet, mit einer Verbindung der Formel VI, worin Z eine Abgangsgruppe bedeutet, kann nach an sich bekannten Methoden durchgeführt werden: Das Amin der Formel V liegt also zweckmässigerweise in der Salzform vor, d.h. die Verbindung der Formel V wird zweckmässigerweise als Alkalimetallsalz, z.B. Lithium-, Natrium- oder Kaliumsalz, oder

als Erdalkalimetallsalz, z.B. Calcium-, Magnesium- oder Bariumsalz, mit einer Verbindung der Formel VI umgesetzt, worin Z für Chlor, Brom, Jod, Mesyloxy oder Tosyloxy steht. Die Umsetzung erfolgt zweckmässigerweise in Gegenwart eines inerten Verdünnungsmittels und bei Temperaturen von etwa -20°C bis 150°C, vorzugsweise im Temperaturbereich von 0°C bis 100°C. Es kommen als Verdünnungsmittel insbesondere inerte organische Lösungsmittel, wie aliphatische oder cyclische Aether, z.B. Diäthyläther, Tetrahydrofuran und Dioxan, Aromaten, z.B. Benzol und Toluol, Dimethylformamid, Dimethylsulfoxid und Hexamethylphosphorsäuretriamid, in Frage. Das Benzamid-Salz der Formel V ist vorzugsweise ein Benzamid-Alkalimetallsalz und die Verbindung der Formel VI vorzugsweise das Bromid.

Im Falle der Umsetzung einer Verbindung der Formel V, worin Y Halogen bedeutet, mit einer Verbindung der Formel VI, worin Z $-NHR^8$ bedeutet, steht Y insbesondere für Chlor oder Brom, wobei Chlor bevorzugt ist. Es handelt sich dabei also um die Umsetzung eines Säurehalogenids mit einem Amin unter Bildung eines Amids. Die Umsetzung erfolgt zweckmässigerweise in Gegenwart eines inerten Verdünnungsmittels, insbesondere eines organischen Lösungsmittels, wie eines aliphatischen oder cyclischen Aethers, z.B. Diäthyläther, Tetrahydrofuran oder Dioxan, eines Aromates, z.B. Benzol oder Toluol, oder eines halogenierten, insbesondere chlorierten, Kohlenwasserstoffes, z.B. Methylenchlorid oder Chloroform, sowie in Gegenwart eines organischen säurebindenden Mittels, wie eines tertiären Amins, z.B. Triäthylamin oder Pyridin. Die Reaktionstemperaturen liegen zweckmässigerweise zwischen -20°C und 100°C, vorzugsweise zwischen 0°C und 20°C.

Die Verfahrensvariante d) oder e) führt zu denjenigen Verbindungen der Formel I, worin A $CR^3$ und $R^4$ Nitro oder Cyano bedeuten bzw. A N oder $CR^3$ bedeutet, wobei im Falle der Verfahrensvariante (e) $R^1$ Trifluormethyl bedeutet, wenn A $CR^3$ und $R^2$ und/oder $R^3$ Halogen darstellen. Das

Symbol "Hal" in den Formeln VIII und X umfasst Fluor, Chlor, Brom und Jod. Die Umsetzung kann zweckmässigerweise durchgeführt werden, indem man das Phenol der Formel VII bzw. das Hydroxybenzoesäurederivat der Formel IX in ein Alkalimetallsalz, beispielsweise unter Verwendung von Natriumhydrid oder Kaliumhydroxid mit azeotroper Wasserentfernung, überführt und anschliessend das Salz mit dem Halogenid der Formel VIII bzw. X reagieren lässt. Die letztgenannte Reaktionsstufe wird zweckmässigerweise in einem dipolaren aprotischen Lösungsmittel, z.B. Dimethylformamid oder Dimethylsulfoxid, oder in Pyridin, bei Temperaturen zwischen 50°C und 200°C, vorzugsweise zwischen 100°C und 180°C, vorgenommen.

Die Nitrierung gemäss der Verfahrensvariante f), die zu denjenigen Verbindungen der Formel I führt, worin $R^4$ Nitro bedeutet, erfolgt zweckmässigerweise mittels Salpetersäure bzw. Salpetersäure enthaltender Gemische oder Lösungen, wie Gemische von Salpetersäure und Schwefelsäure, Lösungen von Salpetersäure in Eisessig und Lösungen von konzentrierter Salpetersäure in chlorierten Kohlenwasserstoffen, z.B. Methylenchlorid, oder mittels Nitroniumtetrafluoroborat, Nitroniumhexafluorophosphat oder Acetylnitrat. Zudem wird zweckmässigerweise bei Temperaturen zwischen 0°C und 200°C, vorzugsweise zwischen 0°C und 25°C, gearbeitet.

Die Isolierung und Reinigung der so hergestellten Verbindungen der Formel I können nach an sich bekannten Methoden erfolgen.

Für den Fall, dass asymmetrische Kohlenstoffatome im Molekül der Formel I vorhanden sind und keine gezielte Synthese zur Isolierung reiner optischer Isomerer durchgeführt wird, fällt das Produkt normalerweise als Racemat an. Die Isomeren können nach an sich bekannten Methoden aufgetrennt werden. Gewünschtenfalls können allerdings

die optischen Isomeren durch Synthese aus entsprechenden optisch aktiven Ausgangsmaterialien hergestellt werden.

Die als Ausgangsmaterialien verwendbaren Verbindungen der Formeln II bis X bzw. Salze und reaktionsfähigen Derivate der Säuren der Formel II und Alkalimetallsalze der Verbindungen der Formeln III, VII und IX sind teilweise bekannt. Das neue N-(5-Fluor-2-nitrobenzoyl)-S,S-dimethyl-sulfoximin (VIII') und das neue N-(5-Hydroxy-2-nitrobenzoyl)-S,S-dimethyl-sulfoximin (IX') bilden einen weiteren Gegenstand der vorliegenden Erfindung. Diese Verbindungen können nach an sich bekannten Methoden hergestellt werden.

Bei den als Ausgangsmaterialien in der Verfahrensvariante f) verwendeten Verbindungen der Formel XI handelt es sich um neue Verbindungen, die ebenfalls einen Aspekt der vorliegenden Erfindung darstellen. Diese Verbindungen, z.B. das N-[3-(o-Chlor-p-trifluormethyl-phenoxy)-benzoyl]-S,S-dimethyl-sulfoximin, können nach Methoden, die analog zu den oben aufgeführten Verfahrensvarianten a) bis e) sind, hergestellt werden, und zwar gemäss folgenden Reaktionsschemata:

Reaktionsschemata :

Herstellung der Verbindungen der allgemeinen Formel XI

a')

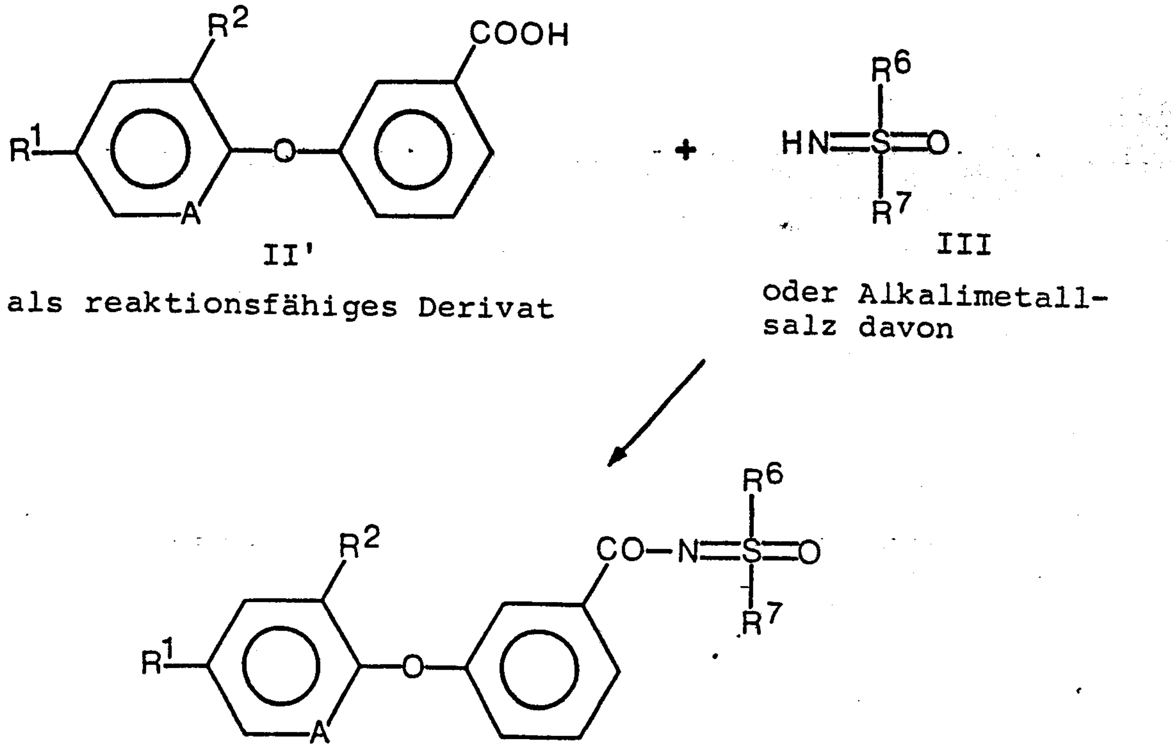

II'

als reaktionsfähiges Derivat

III

oder Alkalimetallsalz davon

b')

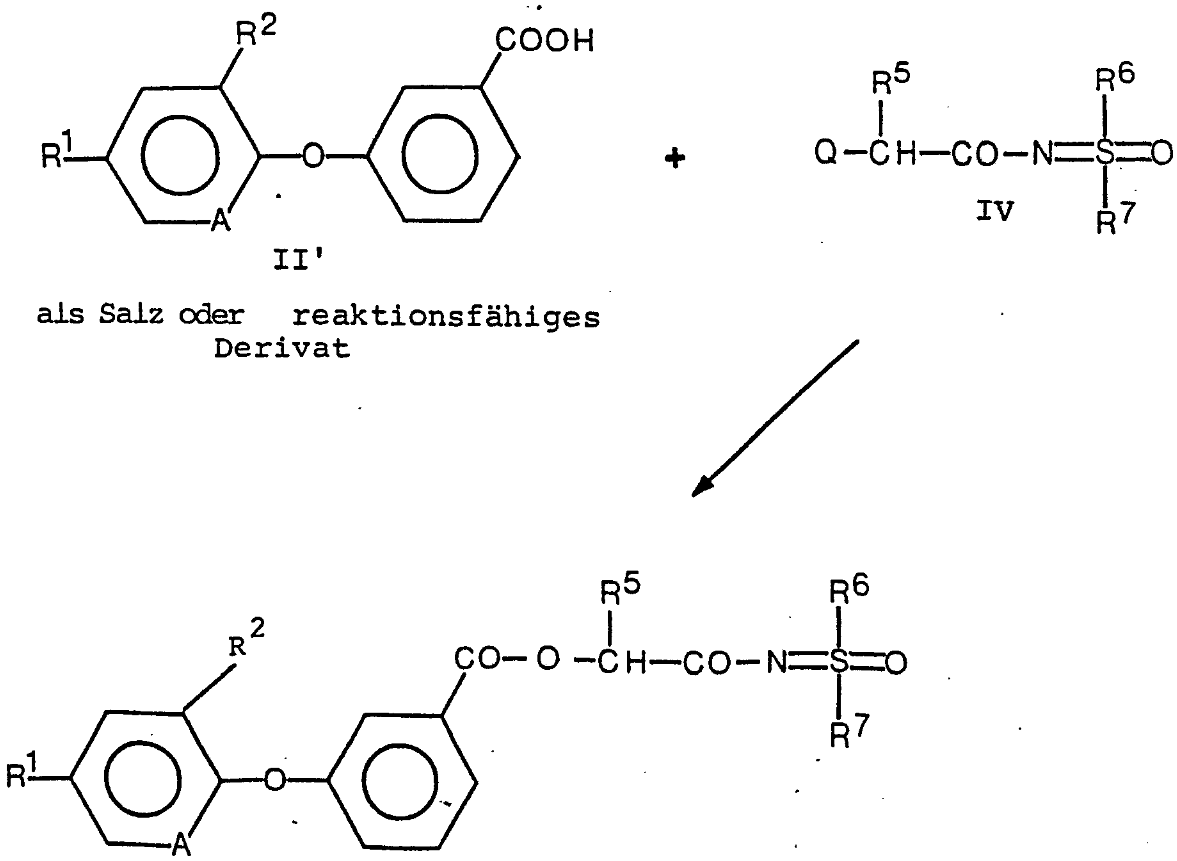

II'

als Salz oder reaktionsfähiges Derivat

IV

c')

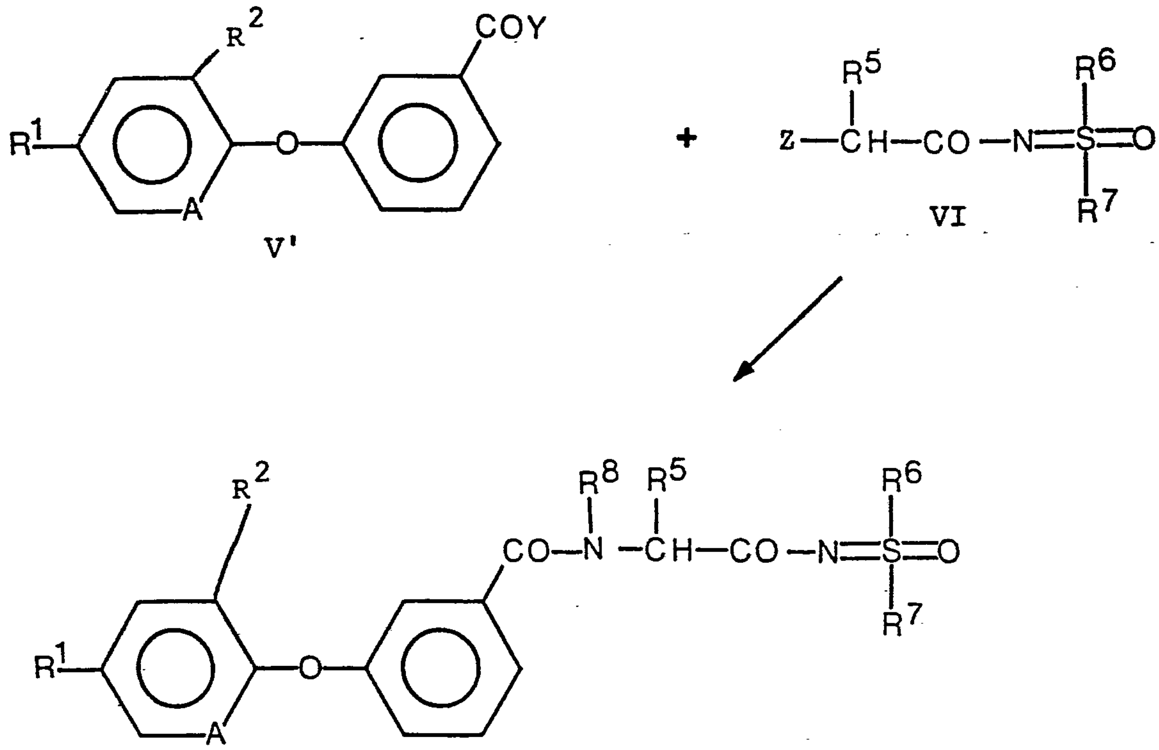

V'

VI

d')

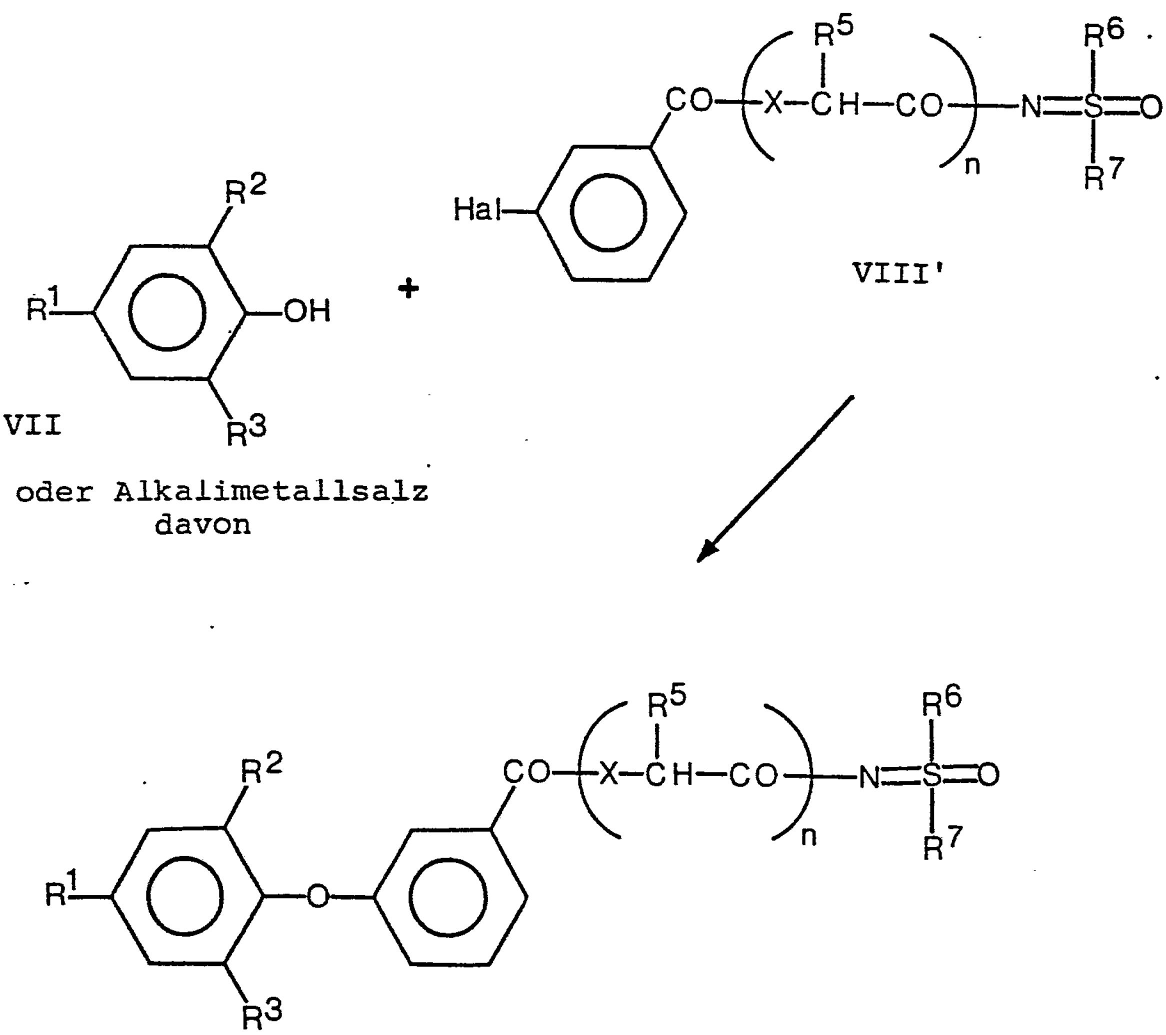

VII

oder Alkalimetallsalz davon

VIII'

e')

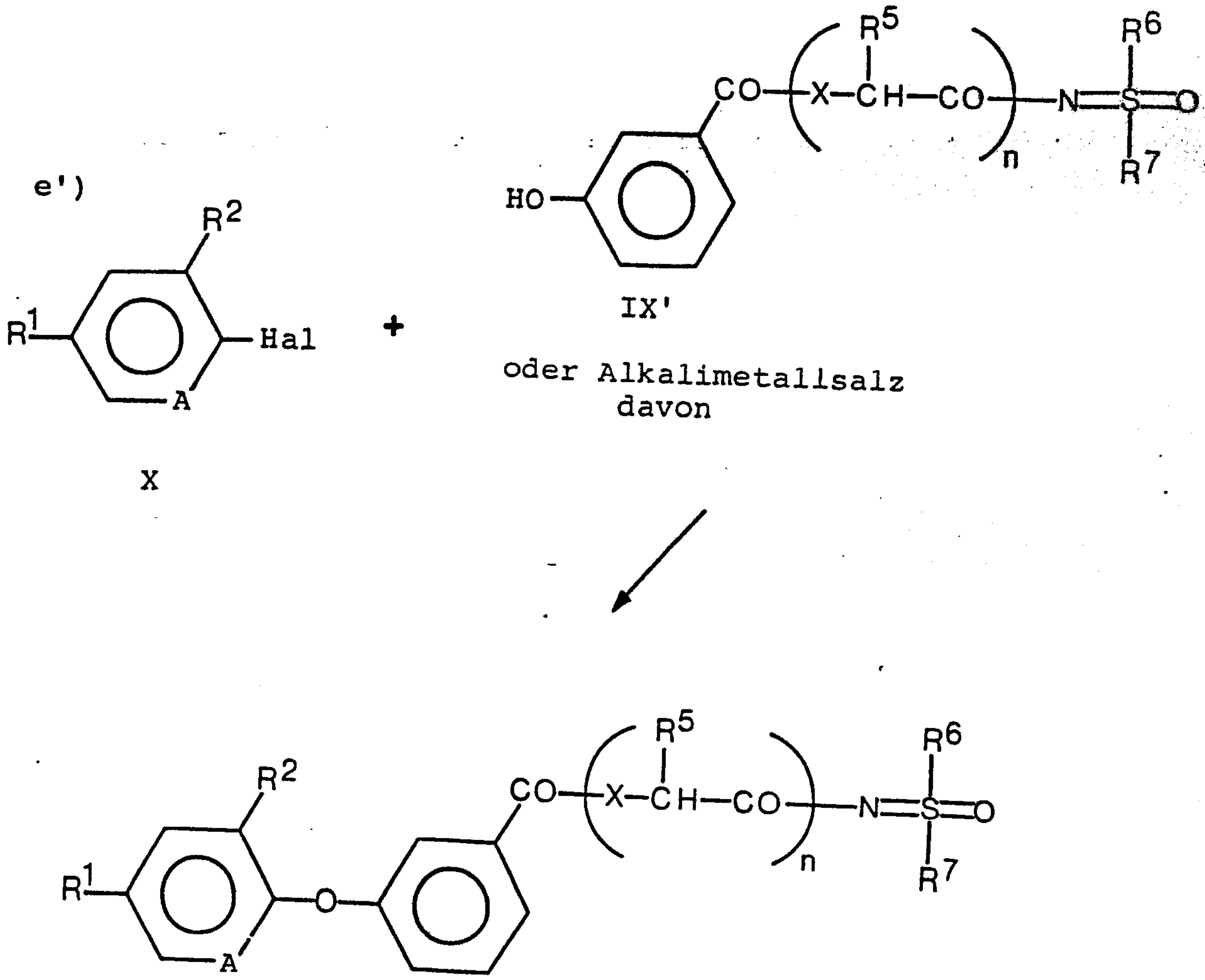

In den obigen Reaktionsschemata besitzen A, $R^1$-$R^8$, n, X, Q, Y, Z und Hal die oben angegebenen Bedeutungen. Die Umsetzungen gemäss den Verfahrensvarianten a') - e') erfolgen zweckmässigerweise unter denjenigen Reaktionsbedingungen, die oben im Zusammenhang mit den entsprechenden Verfahrensvarianten a) - e) angegeben sind. Die als Ausgangsmaterialien verwendbaren Verbindungen der Formeln II', V', VIII', und IX' bzw. Salze und reaktionsfähigen Derivate der Säuren der Formel II' und Alkalimetallsalze der Verbindungen der Formel IX' sind entweder bekannt oder können nach an sich bekannten Methoden hergestellt werden.

Die Verbindungen der Formel I besitzen herbizide Eigenschaften und eignen sich zur Bekämpfung von Unkräutern, insbesondere von Galium aparine, Amaranthus retroflexus, Datura stramonium, Ipomea spp., Abutilon theophrasti, Chenopodium album, Sorghum bicolor, Daucus carota und Cassia obtusifolia in Getreide, insbesondere in Gersten-, Hafer-, Weizen-, Reis-, Erdnuss- und Soyakulturen. Besonders bevorzugt eignen sich die erfindungsgemässen Verbindungen zur Bekämpfung von Unkräutern in Weizen-, Reis-, Erdnuss- und Soyakulturen.

Im allgemeinen genügt eine Konzentration von 0,05 bis 2 kg Wirkstoff der Formel I/ha, vorzugsweise 0,1 bis 1 kg Wirkstoff der Formel I/ha, um den gewünschten herbiziden Effekt zu erzielen.

Die Verbindungen der Formel I sind sowohl Vorauflauf-Herbizide als auch Nachauflauf-Herbizide, wobei ihre Anwendung als Nachauflauf-Herbizide bevorzugt ist.

Das erfindungsgemässe Unkrautbekämpfungsmittel ist dadurch gekennzeichnet, dass es eine wirksame Menge mindestens einer Verbindung der Formel I, wie oben definiert, sowie Formulierungshilfsstoffe enthält. Das Mittel enthält zweckmässigerweise zumindest einen der folgenden

Formulierungshilfsstoffe: feste Trägerstoffe; Lösungs- bzw. Dispersionsmittel; Tenside (Netz- und Emulgiermittel); Dispergatoren (ohne Tensidwirkung); und Stabilisatoren. Unter Verwendung solcher und anderer Hilfsstoffe können die Verbindungen der Formel I, also die herbiziden Wirkstoffe, in die üblichen Formulierungen übergeführt werden, wie Stäube, Pulver, Granulate, Lösungen, Emulsionen, Suspensionen, emulgierbare Konzentrate, Pasten und dergleichen.

Die Verbindungen der Formel I sind im allgemeinen wasserunlöslich und können nach den für wasserunlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln und/oder von Dispergatoren, durch Verdünnen bereits vorbereiteter emulgierbarer Konzentrate mit Lösungs- bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit,Talkum, Attapulgit und Montmorillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs- bzw. Dispersionsmittel kommen im wesentlichen in Frage: Aromaten, wie Benzol, Toluol, Xylole und Alkylnaphthaline; chlorierte Aromaten und chlorierte

aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzol-sulfonat, und Butylnaphthalinsulfonate; und komplexere Fettsulfonate, z.B. die Amidkondensationsprodukte von Oelsäure und N-Methyltaurin und das Natriumsulfonat

von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäuren, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butylhydroxytoluol; UV-Absorber, z.B. substituierte Benzophenone, Diphenylacrylonitrilsäurester und Zimtsäureester; und Deaktivatoren, z.B. Salze der Aethylendiaminotetraessigsäure und Polyglykole.

Die erfindungsgemässen Unkrautbekämpfungsmittel können zusätzlich zu den Verbindungen der Formel I Synergisten und andere Wirkstoffe, z.B. Insektizide, Akarizide, Bakterizide, anderweitige Herbizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0,005 und 95 Gewichtsprozent, vorzugsweise zwischen 5 und 80 Gewichtsprozent einer bzw. mehrerer Verbindungen der Formel I als Wirkstoff(e). Sie können z.B. in einer Form vorliegen, die sich für die Lagerung und den Transport eignet. In solchen Formulierungen, z.B. emulgierbaren Konzentraten, ist die Wirkstoffkonzentration normalerweise im höheren Bereich, vorzugsweise zwischen 10 und 80 Gewichtsprozent, insbesondere zwischen 25 und 75 Gewichtsprozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0,005 bis 2 Gewichtsprozent, insbesondere ca. 0,05 bis 1 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine Verbindung der Formel I, mit festem Trägerstoff vermischt werden, z.B. durch Zusammenmahlen; oder man kann den festen Trägerstoff mit einer Lösung oder Suspension des Wirkstoffes imprägnieren und dann das Lösungs- bzw. Dispersionsmittel durch Abdunsten, Erhitzen oder Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die Verbindung der Formel I kann auch mit einem Tensid und einem festen Trägerstoff zur Bildung eines netzbaren Pulvers vermischt werden, welches in Wasser dispergierbar ist, oder sie kann mit einem festen vorgranulierten Trägerstoff zur Bildung eines granulatförmigen Produktes

vermischt werden.

Wenn gewünscht, kann die Verbindung der Formel I in einem mit Wasser nicht mischbaren Lösungsmittel, wie beispielsweise einem hochsiedenden Kohlenwasserstoff, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einer erfindungsgemässen Verbindung bzw. einem erfindungsgemässen Unkrautbekämpfungsmittel behandelt.

Die nachfolgenden Beispiele dienen zur näheren Erläuterung der Erfindung.

## I. Herstellung der Wirkstoffe der Formel I:

### Beispiel 1

Zu einer Lösung von 36,14 g 5-(o-Chlor-p-trifluormethyl-phenoxy)-2-nitro-benzoesäure in 1,5 l Methylenchlorid wird bei 5-10°C unter Rühren eine Lösung von 20,63 g Dicyclohexylcarbodiimid in 100 ml Methylenchlorid während 10 Minuten zugetropft. Anschliessend wird zur Suspension bei 20°C eine Lösung von 9,31 g Dimethylsulfoximin in 50 ml Methylenchlorid während 5 Minuten zugetropft, und das Reaktionsgemisch wird 20 Stunden bei Raumtemperatur nachgerührt. Man nutscht den gebildeten, unlöslichen Dicyclohexylharnstoff ab und dampft das Filtrat unter vermindertem Druck ein. Der Rückstand wird an Kieselgel mit Diäthyläther chromatographisch gereinigt.

Man erhält das N-[5-(o-Chlor-p-trifluormethyl-phenoxy)-2-nitrobenzoyl]-S,S-dimethyl-sulfoximin als farblose Kristalle, Smp. 126-127°C; $^1$H-NMR ($CDCl_3$): 7,99 (d,1H), 7,80 (d,1H), 7,60 (q,1H), 7,23 (d,1H), 7,14 (d,1H), 7,02 (q,1H) und 3,40 (s,6H).

In analoger Weise erhält man ausgehend von

5-(o-Chlor-p-trifluormethyl-phenoxy)-2-nitro-benzoesäure und Aethylmethylsulfoximin das N-[5-(o-Chlor-p-trifluormethyl-phenoxy)-2-nitrobenzoyl]-S-äthyl-S-methyl-sulfoximin als farbloses zähes Oel; $^1$H-NMR ($CDCl_3$): 7,96 (d,1H), 7,80 (d,1H), 7,59 (q,1H), 7,22 (d,1H), 7,16 (d,1H), 7,01 (q,1H), 3,48 (m,2H), 3,37 (s,3H) und 1,5 (t,3H).

5-(o-Chlor-p-trifluormethyl-phenoxy)-2-nitro-benzoesäure und Diäthylsulfoximin das N-[5-(o-Chlor-p-trifluormethyl-phenoxy)-2-nitrobenzoyl]-S,S-diäthyl-sulfoximin als hellgelbes zähes Harz; $^1$H-NMR ($CDCl_3$): 7,92 (d,1H), 7,80 (d,1H), 7,58 (q,1H), 7,21 (d,1H), 7,20 (d,1H), 7,00 (q,1H), 3,52 (m,4H) und 1,49 (t,6H).

5-(o-Chlor-p-trifluormethyl-phenoxy)-2-nitro-benzoesäure und Methylphenylsulfoximin das N-[5-(o-Chlor-p-trifluormethyl-phenoxy)-2-nitrobenzoyl]-S-methyl-S-phenyl-sulfoximin als hellgelbes zähes Harz; $^{1}$H-NMR ($CDCl_3$): 8,02 (m,2H), 7,97 (d,1H), 7,79 (d,1H), 7,71 (m,1H), 7,63 (m,2H), 7,58 (q,1H), 7,21 (d,1H), 7,19 (d,1H), 7,04 (q,1H) und 3,53 (s,3H).

5-(o-Chlor-p-trifluormethyl-phenoxy)-2-nitro-benzoesäure und (n-Hexyl)-methyl-sulfoximin das N-[5-(o-Chlor-p-trifluormethyl-phenoxy)-2-nitrobenzoyl]-S-(n-hexyl)-S-methyl-sulfoximin als gelbes zähes Harz; $^{1}$H-NMR ($CDCl_3$): 8,1-6,9 (m,6H), 3,7-3,3 (m,5H) und 2,2-0,8 (m,11H).

5-(o-Chlor-p-trifluormethyl-phenoxy)-2-nitro-benzoesäure und Methyl-(p-chlorphenyl)-sulfoximin das N-[5-(o-Chlor-p-trifluormethyl-phenoxy)-2-nitrobenzoyl]-S-methyl-S-(p-chlorphenyl)-sulfoximin als hellgelbes Harz; $^{1}$H-NMR ($CDCl_3$): 7,99-7,92 (m,3H), 7,79 (d,1H), 7,65-7,55 (m,3H), 7,22 (d,1H), 7,18 (d,1H), 7,05 (q,1H), 3,50 (s,3H).

5-(o-Chlor-p-trifluormethyl-phenoxy)-2-nitro-benzoesäure und Methyl-(p-tolyl)-sulfoximin das N-[5-(o-Chlor-p-trifluormethyl-phenoxy)-2-nitrobenzoyl]-S-methyl-S-(p-tolyl)-sulfoximin als gelbes Harz; $^{1}$H-NMR ($CDCl_3$): 7,95 (d,1H), 7,89 (m,2H), 7,79 (d,1H), 7,58 (q,1H), 7,47 (m,2H), 7,22 (d,1H), 7,19 (d,1H), 7,05 (q,1H), 3,50 (s,3H), 2,47 (s,3H).

5-(o-Chlor-p-trifluormethyl-phenoxy)-2-nitro-benzoesäure und Methyl-(o-chlorphenyl)-sulfoximin das N-[5-(o-Chlor-p-trifluormethyl-phenoxy)-2-nitrobenzoyl]-S-methyl-S-(o-chlorphenyl)-sulfoximin als helles Harz; $^{1}$H-NMR ($CDCl_3$): 8,28 (q,1H), 7,95 (d,1H), 7,79 (d,1H), 7,68-7,52 (m,4H), 7,22 (d,1H), 7,20 (d,1H), 7,02 (q,1H), 3,68 (s,3H).

5-(5-Trifluormethyl-2-pyridyloxy)-2-nitro-benzoesäure und Dimethylsulfoximin das N-[5-(5-Trifluormethyl-2-pyridyloxy)-2-nitrobenzoyl]-S,S-dimethyl-sulfoximin als gelbes Harz; $^{1}H$-NMR ($CDCl_3$): 8,45 (d,1H), 8,02-7,94 (m,2H), 7,50 (d,1H), 7,32 (q,1H), 7,13 (d,1H), 3,40 (s,6H).

5-(3,5-Dichlor-2-pyridyloxy)-2-nitro-benzoesäure und Dimethylsulfoximin das N-[5-(3,5-Dichlor-2-pyridyloxy)-2-nitrobenzoyl]-S,S-dimethyl-sulfoximin als gelbes Harz; $^{1}H$-NMR ($CDCl_3$): 8,02 (d,1H), 7,98 (d,1H), 7,83 (d,1H), 7,47 (d,1H), 7,29 (q,1H), 3,40 (s,6H).

5-(3-Chlor-5-trifluormethyl-2-pyridyloxy)-2-nitrobenzoesäure und Dimethylsulfoximin das N-[5-(3-Chlor-5-trifluormethyl-2-pyridyloxy)-2-nitrobenzoyl]-S,S-dimethylsulfoximin als helles Harz; $^{1}H$-NMR ($CDCl_3$): 8,30 (d,1H), 8,04 (d,1H), 7,99 (d,1H), 7,55 (d,1H), 7,35 (q,1H), 3,40 (s,6H).

## Beispiel 2

Eine Lösung von 2,46 g 2-Chlor-5-(o-chlor-p-trifluormethyl-phenoxy)-benzoesäure in 20 ml Methylenchlorid wird bei Raumtemperatur unter Rühren zu einer Lösung von 1,3 g Dicyclohexylcarbodiimid und 0,6 g Dimethylsulfoximin in 5 ml Methylenchlorid zugetropft, wobei eine schwach exotherme Reaktion stattfindet. Nach 15-minütiger Reaktionszeit wird das Lösungsmittel unter vermindertem Druck abgedampft und der Rückstand an Kieselgel mit n-Hexan/Aethylacetat (9:1) chromatographisch gereinigt. Das weisse, kristalline Produkt wird dann aus Methylenchlorid/n-Hexan umkristallisiert. Man erhält das N-[2-Chlor-5-(o-chlor-p-trifluormethyl-phenoxy)-benzoyl]-S,S-dimethyl-sulfoximin, Smp. 128-131°C; Massenspektrum: m/e 425/427; $^{1}H$-NMR ($CDCl_3$): 7,8-6,9 (m,6H), 3,4 (s,6H).

In analoger Weise erhält man ausgehend von

5-(o-Chlor-p-trifluormethyl-phenoxy)-2-jodbenzoesäure und Dimethylsulfoximin das N-[5-(o-Chlor-p-trifluormethyl-phenoxy)-2-jodbenzoyl]-S,S-dimethyl-sulfoximin, Smp. 88-91°C; Massenspektrum: m/e 517/519; $^1$H-NMR ($CDCl_3$): 8,0-6,6 (m,6H), 3,4 (s,6H).

Beispiel 3

Zu einer Lösung von 9,31 g Dimethylsulfoximin in 375 ml absolutem 1,2-Dimethoxyäthan wird bei 25°C unter Rühren 50,40 g einer 25%igen Lösung von Kalium-tert.-pentylat in Toluol während 30 Minuten zugetropft. Die Suspension wird unter vermindertem Druck zur Trockene eingedampft. Auf diese Weise erhält man das Ausgangsmaterial Dimethylsulfoximin-Kaliumsalz.

Zu einer Suspension des Dimethylsulfoximin-Kaliumsalzes in 250 ml absolutem 1,2-Dimethoxyäthan wird bei 10-15°C unter Rühren eine Lösung von 38,01 g 5-(o-Chlor-p-trifluormethyl-phenoxy)-2-nitrobenzoylchlorid in 125 ml absolutem 1,2-Dimethoxyäthan während 30 Minuten zugetropft. Das Reaktionsgemisch wird 24 Stunden bei Raumtemperatur nachgerührt und anschliessend unter vermindertem Druck zur Trockene eingedampft. Der harzige Rückstand wird in 1,25 l Diäthyläther gelöst, die Lösung durch Celite filtriert und das Filtrat zur Trockene eingedampft. Anschliessend wird der Rückstand an Kieselgel mit Methylenchlorid chromatographisch gereinigt. Man erhält das N-[5-(o-Chlor-p-trifluormethyl-phenoxy)-2-nitrobenzoyl]-S,S-dimethyl-sulfoximin als farblose Kristalle, Smp. 126-127°C; $^1$H-NMR ($CDCl_3$): 7,99 (d,1H), 7,80 (d,1H), 7,60 (q,1H), 7,23 (d,1H), 7,14 (d,1H), 7,02 (q,1H) und 3,40 (s,6H).

In analoger Weise erhält man ausgehend von

Tetramethylensulfoximin-Kaliumsalz und 5-(o-Chlor-p-trifluormethyl-phenoxy)-2-nitrobenzoylchlorid das N-[5-(o-Chlor-p-trifluormethyl-phenoxy)-2-nitrobenzoyl]-S,S-tetramethylen-sulfoximin als gelbes Harz; $^{1}$H-NMR ($CDCl_3$): 8,00 (d,1H), 7,80 (d,1H), 7,60 (q,1H), 7,24 (d,1H), 7,14 (d,1H), 7,03 (q,1H), 3,77 (m,2H), 3,32 (m,2H), 2,36 (m,4H).

## Beispiel 4

Eine Lösung von 4,5 g 5-(o-Chlor-p-trifluormethyl-phenoxy)-2-nitro-benzoesäure in 50 ml Thionylchlorid wird während 1 Stunde bei Rückflusstemperatur erhitzt und danach zur Trockene eingedampft. Es wird auf diese Weise das rohe Säurechlorid hergestellt.

Zu einer Lösung des rohen Säurechlorids (4,9 g) in 20 ml Methylenchlorid wird eine Lösung von 2,5 g S-Methyl-S-(p-nitrophenyl)-sulfoximin und 0,99 g Pyridin in 15 ml Methylenchlorid während 10 Minuten zugetropft, wobei die Temperatur des Reaktionsgemisches von 23°C auf 27°C ansteigt. Nach beendeter Zugabe wird das Gemisch 30 Minuten bei Raumtemperatur nachgerührt. Anschliessend giesst man das Gemisch auf 100 ml Wasser, säuert mit konzentrierter Salzsäure auf pH 1 an und extrahiert die wässrige Phase zweimal mit jeweils 150 ml Aethylacetat. Die vereinigten organischen Phasen werden zweimal mit jeweils 100 ml Kochsalzlösung gewaschen, über wasserfreiem Natriumsulfat getrocknet und zur Trockene eingedampft. Das resultierende dickflüssige orange Oel (8,2 g) wird an Kieselgel (Ø = 0,063 mm; 400 g) mit Aethylacetat/Diäthyläther (1:1) chromatographisch gereinigt. Man erhält 5,0 g (73,5% der theoretischen Ausbeute) N-[5-(o-Chlor-p-trifluormethyl-phenoxy)-2-nitrobenzoyl]-S-methyl-S-(p-nitrophenyl)-sulfoximin, Smp. 62-63°C; $^{1}$H-NMR ($CDCl_3$): 8,48 (d,2H), 8,257 (d,2H), 7,965 (d,1H), 7,795 (d,1H), 7,595 (doppel-d,1H), 7,235 (d,1H), 7,182 (d,1H), 7,055 (doppel-d,1H), 3,52 (s,3H).

In analoger Weise erhält man ausgehend von

5-(o-Chlor-p-trifluormethyl-phenoxy)-2-nitrobenzoesäurechlorid und S-Methyl-S-(p-methoxyphenyl)-sulfoximin

das N-[5-(o-Chlor-p-trifluormethyl-phenoxy)-2-nitrobenzoyl]-S-methyl-S-(p-methoxyphenyl)-sulfoximin, Smp. 135-137°C; $^1$H-NMR ($CDCl_3$): 7,96 (d,1H), 7,92 (d,2H), 7,79 (d,1H), 7,58 (doppel-d,1H), 7,21 (d,1H), 7,19 (d,1H), 7,075 (d,2H), 7,035 (doppel-d,1H), 3,89 (s,3H), 3,50 (s,3H).

## Beispiel 5

Zu einer Lösung von 3,61 g 5-(o-Chlor-p-trifluormethyl-phenoxy)-2-nitro-benzoesäure und 1,21 g Triäthylamin in 30 ml absolutem Aceton wird unter Rühren während 30 Minuten bei -5°C eine Lösung von 1,29 g Chlorameisensäure-äthylester in 10 ml absolutem Aceton zugetropft. Das Reaktionsgemisch wird 2 Stunden bei Raumtemperatur nachgerührt, dann wird eine Lösung von 1,42 g S,S-Tetramethylensulfoximin in 5 ml absolutem Aceton während 5 Minuten zugetropft. Man rührt das Reaktionsgemisch 2 Stunden bei Raumtemperatur nach, dampft es unter vermindertem Druck ein, löst den Rückstand in Diäthyläther und wäscht die Lösung mit Wasser. Die organische Phase wird dann getrocknet und zur Trockene eingedampft und der Rückstand an Kieselgel mit Diäthyläther/n-Hexan (3:1) chromatographisch gereinigt.

Man erhält das N-[5-(o-Chlor-p-trifluormethyl-phenoxy)-2-nitrobenzoyl]-S,S-tetramethylen-sulfoximin als gelbes Harz; $^1$H-NMR ($CDCl_3$): 8,00 (d,1H), 7,80 (d,1H), 7,60 (q,1H), 7,24 (d,1H), 7,14 (d,1H), 7,03 (q,1H), 3,77 (m,2H), 3,32 (m,2H), 2,36 (m,4H).

## Beispiel 6

Eine Lösung von 3,1 g 2-Brompropionsäure in 20 ml Methylenchlorid wird mit einer Lösung von 4,2 g Dicyclohexylcarbodiimid in 10 ml Methylenchlorid versetzt. Anschliessend wird bei 10°C eine Lösung von 3,1 g Methylphenylsulfoximin in 10 ml Methylenchlorid zugegeben, und das Reaktionsgemisch wird 5 Stunden bei 20°C gerührt. Man nutscht den gebildeten, unlöslichen Dicyclohexylharnstoff ab und dampft das Filtrat unter vermindertem Druck ein. Man erhält das N-(2-Brompropionyl)-S-methyl-S-phenyl-sulfoximin, Smp. 109-110°C, das für die Endstufe des Herstellungsverfahrens (folgend) benötigt wird.

0,25 g Natriumhydrid werden zu einer Lösung von 3,6 g 5-(o-Chlor-p-trifluormethyl-phenoxy)-2-nitrobenzoesäure in 10 ml Dimethylformamid zugegeben, und das so hergestellte Natriumsalz der Säure wird mit einer Lösung von 2,9 g N-(2-Brompropionyl)-S-methyl-S-phenyl-sulfoximin in 5 ml Dimethylformamid versetzt. Anschliessend wird das Reaktionsgemisch während 4 Stunden bei 60°C gerührt. Man nutscht das gebildete Natriumbromid ab, dampft das Filtrat unter vermindertem Druck ein und reinigt den Rückstand chromatographisch an Kieselgel mit Aethylacetat. Auf diese Weise erhält man das N-{2-[5-(o-Chlor-p-trifluormethyl-phenoxy)-2-nitrobenzoyloxy]-propionyl}-S-methyl-S-phenyl-sulfoximin als gelbe Flüssigkeit; $^1$H-NMR ($CDCl_3$): 8,05-7,96 (m,3H), 7,8 (doppel-d,1H), 7,7-7,55 (m,4H), 7,35 (doppel-d,1H), 7,2 (doppel-d,1H), 7,0 (m,1H), 5,41 und 5,35 (je d,1H), 3,36 (s,3H), 1,62 und 1,61 (je d,3H).

In analoger Weise erhält man ausgehend von

2-Brompropionsäure, Aethylmethylsulfoximin und 5-(o-Chlor-p-trifluormethyl-phenoxy)-2-nitrobenzoesäure das N-{2-[5-(o-Chlor-p-trifluormethyl-phenoxy)-2-nitrobenzoyloxy]-propionyl}-S-äthyl-S-methyl-sulfoximin als gelbe Flüssigkeit; $^1$H-NMR ($CDCl_3$): 8,02 (d,1H), 7,81 (d,1H), 7,61 (doppel-d,1H), 7,37 (doppel-d,1H), 7,23 (d,1H), 7,03 (m,1H), 5,29 (doppel-q,1H), 3,52-3,38 (m,2H), 3,24 und 3,23 (je s,3H) und 1,59 (d,3H).

2-Brompropionsäure, Methyl-(p-chlorphenyl)-sulfoximin und 5-(o-Chlor-p-trifluormethyl-phenoxy)-2-nitrobenzoesäure das N-{2-[5-(o-Chlor-p-trifluormethyl-phenoxy)-2-nitrobenzoyloxy]-propionyl}-S-methyl-S-(p-chlorphenyl)-sulfoximin

als gelbes Harz; $^1$H-NMR ($CDCl_3$): 8,02 und 7,99 (doppel-d,1H), 7,98-7,89 (m,2H), 7,82 und 7,79 (doppel-d,1H), 7,63-7,54 (m,3H), 7,37 und 7,34 (doppel-d,1H), 7,23 und 7,19 (doppel-d, 1H), 7,02 und 6,99 (doppel-q,1H), 5,37 und 5,31 (doppel-q, 1H), 3,36 und 3,34 (doppel-s,3H), 1,62 und 1,60 (doppel-d, 3H).

2-Brompropionsäure, Methyl-(p-tolyl)-sulfoximin und 5-(o-Chlor-p-trifluormethyl-phenoxy)-2-nitrobenzoesäure das N-{2-[5-(o-Chlor-p-trifluormethyl-phenoxy)-2-nitro-benzoyloxy]-propionyl}-S-methyl-S-(p-tolyl)-sulfoximin als gelbes Harz; $^1$H-NMR ($CDCl_3$): 8,02 und 8,00 (doppel-d,1H), 7,90-7,82 (m,2H), 7,80 und 7,79 (doppel-d,1H), 7,60 und 7,57 (doppel-q,1H), 7,44-7,34 (m,3H), 7,23 und 7,18 (doppel-d,1H), 7,02 und 6,99 (doppel-q,1H), 5,40 und 5,35 (doppel-q, 1H), 3,35 und 3,34 (doppel-s,3H), 2,46 und 2,44 (doppel-s, 1H), 1,63 und 1,61 (doppel-d,3H).

2-Brompropionsäure, S,S-Tetramethylensulfoximin und 5-(o-Chlor-p-trifluormethyl-phenoxy)-2-nitrobenzoesäure das N-{2-[5-(o-Chlor-p-trifluormethyl-phenoxy)-2-nitro-benzoyloxy]-propionyl}-S,S-tetramethylen-sulfoximin als gelbes Harz; $^1$H-NMR ($CDCl_3$): 8,01 (d,1H), 7,81 (d,1H), 7,61 (q,1H), 7,37 (d,1H), 7,24 (d,1H), 7,03 (q,1H), 5,32 (q,1H), 3,65-3,54 (m,2H), 3,32-3,21 (m,2H), 2,40-2,22 (m,4H), 1,60 (d,3H).

Chloressigsäure, Dimethylsulfoximin und 5-(o-Chlor-p-trifluormethyl-phenoxy)-2-nitrobenzoesäure unter Verwendung von 1,5-Diaza-bicyclo[5,4,0]undec-5-en als Base und von Toluol als Lösungsmittel das N-{[5-(o-Chlor-p-trifluormethyl-phenoxy)-2-nitrobenzoyloxy]acetyl}-S,S-dimethyl-sulfoximin als gelbes Harz; $^1$H-NMR ($CDCl_3$): 8,23-6,92 (m,6H), 4,87 (s,2H), 3,37 (s,6H).

2-Brompropionsäure, Dimethylsulfoximin und 5-(o-Chlor-p-trifluormethyl-phenoxy)-2-nitrobenzoesäure unter Verwendung von Natriummethylat als Base und von 1,2-Dimethoxyäthan als Lösungsmittel das N-{2-[5-(o-Chlor-p-trifluormethyl-phenoxy)-2-nitrobenzoyloxy]-propionyl}-S,S-dimethyl-sulfoximin als gelbes Harz; $^1$H-NMR ($CDCl_3$): 8,20-6,90 (m,6H), 5,28 (q,1H), 3,37 (s,6H), 1,62 (d,3H).

2-Brompropionsäure, S-Methyl-S-(p-methoxyphenyl)-sulfoximin und 5-(o-Chlor-p-trifluormethyl-phenoxy)-2-nitrobenzoesäure das N-{2-[5-(o-Chlor-p-trifluormethyl-phenoxy)-2-nitrobenzoyloxy]-propionyl}-S-methyl-S-(p-methoxyphenyl)-sulfoximin; Smp. 48-58°C; $^1$H-NMR ($CDCl_3$): 8,20 und 7,995 (doppel-d,2H), 7,92 und 7,89 (doppel-d,2H), 7,81 und 7,79 (doppel-d,1H), 7,60 und 7,58 (2x doppel-d,1H), 7,395 und 7,35 (doppel-d,1H), 7,22 und 7,18 (doppel-d,1H), 7,06 und 7,03 (doppel-d,2H), 7,01 und 6,98 (2x doppel-d,1H), 5,40 und 5,35 (doppel-q,1H), 3,885 und 3,87 (doppel-s,3H), 3,35 und 3,336 (doppel-s,3H), 1,63 und 1,605 (doppel-d,3H).

2-Brompropionsäure, S-Methyl-S-(p-nitrophenyl)-sulfoximin und 5-(o-Chlor-p-trifluormethyl-phenoxy)-2-nitrobenzoesäure das N-{2-[5-(o-Chlor-p-trifluormethyl-phenoxy)-2-nitrobenzoyloxy]-propionyl}-S-methyl-S-(p-nitrophenyl)-sulfoximin; Smp. 58-64°C; $^1$H-NMR ($CDCl_3$): 7,95 und 7,925 (doppel-d,2H), 7,725 und 7,70 (doppel-d,2H), 7,515 und 7,48 (doppel-d,1H), 7,315 und 7,285 (doppel-d,1H), 7,12 und 7,088 (2x doppel-d,1H), 6,885 und 6,80 (doppel-d,1H), 6,75 und 6,697 (doppel-d,1H), 6,535 und 6,48 (2x doppel-d, 1H), 5,35 und 5,250 (doppel-q,1H), 4,01 und 3,375 (doppel-s, 3H), 1,61 und 1,60 (doppel-d,3H).

## Beispiel 7

0,48 g Natriumhydrid werden in 10 ml absolutes Dimethylformamid vorgelegt. Man tropft 1,96 g o-Chlor-p-trifluormethyl-phenol zu und rührt das Gemisch 1 Stunde nach. Anschliessend gibt man 2,6 g N-(5-Fluor-2-nitro-benzoyl)-S,S-dimethyl-sulfoximid zu und erhitzt während 90 Minuten auf 100°C. Man kühlt danach das Reaktionsgemisch ab, giesst es auf 100 ml Eiswasser und extrahiert mit 50 ml

Aethylacetat. Die Essigesterphase wird mit Wasser gewaschen, getrocknet und eingedampft, und der Rückstand wird chromatographisch gereinigt und aus Diäthyläther kristallisiert. Man isoliert 1 g N-[5-(o-Chlor-p-trifluormethyl-phenoxy)-2-nitrobenzoyl]-S,S-dimethyl-sulfoximin, Smp. 122°C.

Das als Ausgangsmaterial verwendete N-(5-Fluor-2-nitrobenzoyl)-S,S-dimethyl-sulfoximin kann wie folgt hergestellt werden:

Eine Lösung von 26,8 g 5-Fluor-2-nitrobenzoesäure in 50 ml Thionylchlorid wird 1 Stunde bei Rückflusstemperatur erhitzt. Das überschüssige Thionylchlorid wird dann abdestilliert und der Rückstand in 50 ml Methylenchlorid gelöst. Bei 0°C tropft man eine Lösung von 13,5 g Dimethylsulfoximin in 11,5 g Pyridin zu und rührt das Gemisch 1 Stunde nach. Anschliessend dampft man das Gemisch ein und reinigt den Rückstand chromatographisch mittels der zehnfachen Menge Kieselgel. Das Produkt wird aus Toluol kristallisiert. Auf diese Weise erhält man N-(5-Fluor-2-nitrobenzoyl)-S,S-dimethyl-sulfoximin, Smp. 113-115°C.

## Beispiel 8

0,14 g Natriumhydrid wird in 20 ml Dimethylsulfoxid vorgelegt. Man tropft eine Lösung von 1,55 g N-(5-Hydroxy-2-nitrobenzoyl)-S,S-dimethyl-sulfoximin in 10 ml DMSO zu und rührt das Gemisch 3 Stunden bei 40°C nach. Anschliessend wird zum Gemisch 1,0 g 2-Fluor-5-trifluormethyl-pyridin zugegeben, und das Reaktionsgemisch wird 16 Stunden bei 70°C erhitzt.

Zur Aufarbeitung wird das abgekühlte Gemisch auf 100 ml 2n Natriumhydroxid gegossen und das wässrige Gemisch dreimal mit jeweils 50 ml Aethylacetat extrahiert. Die vereinigten organischen Phasen werden zweimal mit jeweils 100 ml Wasser nachgewaschen, getrocknet und eingedampft,

und der Rückstand wird mit n-Hexan/Aethylacetat (9:1) an Kieselgel chromatographisch gereinigt.

Das so erhaltene Produkt, N-[5-(5-Trifluormethyl-2-pyridyloxy)-2-nitrobenzoyl]-S,S-dimethyl-sulfoximin, weist die gleichen physikalischen Angaben wie die in Beispiel 1 entsprechende Verbindung I (9. Endprodukt) auf.

Das als Ausgangsmaterial verwendete N-(5-Hydroxy-2-nitrobenzoyl)-S,S-dimethyl-sulfoximin kann wie folgt hergestellt werden:

Zu einer Suspension von 22,5 g 5-Acetoxy-2-nitrobenzoesäure in 500 ml Methylenchlorid wird bei Raumtemperatur unter Rühren eine Lösung von 20,6 g Dicyclohexylcarbodiimid in 100 ml Methylenchlorid während 10 Minuten zugetropft. Anschliessend wird zum Reaktionsgemisch eine Lösung von 9,3 g Dimethylsulfoximin in 50 ml Methylenchlorid während 5 Minuten zugetropft, und das Gemisch wird 3 Stunden bei Raumtemperatur nachgerührt. Man nutscht den gebildeten, unlöslichen Dicyclohexylharnstoff ab und dampft das Filtrat unter vermindertem Druck ein. Der Rückstand wird an Kieselgel mit Methylenchlorid/Aethylacetat (1:1) chromatographisch gereinigt.

Man erhält das N-(5-Acetoxy-2-nitrobenzoyl)-S,S-dimethyl-sulfoximin als dickflüssiges, gelbes Oel; $^{1}H$-NMR ($CDCl_3$): 7,94 (d,1H), 7,50 (d,1H), 7,28 (q,1H), 3,40 (s,6H), 2,36 (t,3H).

Eine Lösung von 30,0 g N-(5-Acetoxy-2-nitrobenzoyl)-S,S-dimethyl-sulfoximin in 500 ml Methanol wird mit 150 ml 2n Natriumhydroxid versetzt, und das Gemisch wird während 20 Minuten bei Raumtemperatur gerührt. Dann wird die resultierende Lösung mit 100 ml 2n Salzsäure auf ca. pH 4 gestellt und das Methanol unter vermindertem Druck abdestilliert. Der Rückstand wird mit Natriumchlorid-Lösung gesättigt und mit Aethylacetat ausgeschüttelt,und die organische Phase

wird getrocknet und eingedampft. Dann wird der kristalline Rückstand aus heissem Aethylacetat umkristallisiert. Man erhält das N-(5-Hydroxy-2-nitrobenzoyl)-S,S-dimethyl-sulfoximin als gelbe Kristalle, Smp. 146-149°C; $^{1}$H-NMR (DMSO): 7,90 (d,1H), 6,95 (d,1H), 6,90 (q,1H), 3,44 (s,6H).

## Beispiel 9

Zu einer Lösung von 195,9 g N-[3-(o-Chlor-p-trifluormethyl-phenoxy)-benzoyl]-S,S-dimethyl-sulfoximin in 750 ml 1,2-Dichloräthan werden während 10 Minuten 400 ml 94%ige Schwefelsäure zugetropft. Das Reaktionsgemisch wird unter Rühren auf -5°C abgekühlt und während 30 Minuten tropfenweise mit einer Lösung von 50,55 g Kaliumnitrat in 400 ml 94%iger Schwefelsäure versetzt. Das Rühren bei -5°C wird noch 15 Minuten fortgesetzt. Anschliessend wird das Gemisch auf 3,5 kg Eis gegossen und das sich ausscheidende Oel dreimal mit jeweils 1,5 l Methylenchlorid extrahiert. Die vereinigten Extrakte werden viermal mit jeweils 750 ml Wasser neutral gewaschen, über wasserfreiem Natriumsulfat getrocknet und unter vermindertem Druck zur Trockene eingedampft. Nach Lösen des Rückstandes in 750 ml Diäthyläther wird die Lösung geimpft und mit 250 ml n-Hexan versetzt. Danach werden die resultierenden Kristalle abgenutscht, zweimal mit jeweils 250 ml Diäthyläther gewaschen und getrocknet, wobei 155,5 g Produkt erhalten werden. Durch Eindampfen der Mutterlauge zur Trockene und Reinigen des Rückstandes (65,0 g) mit Diäthyläther an einer Säule aus 1,25 kg Kieselgel 60 erhält man weitere 14,2 g Produkt in Form von gelben Kristallen. Das gesamte Produkt besteht also aus 169,7 g (77,7% der theoretischen Ausbeute) N-[5-(o-Chlor-p-trifluormethyl-phenoxy)-2-nitrobenzoyl]-S,S-dimethyl-sulfoximin, Smp. 118-125°C.

Das als Ausgangsmaterial verwendete N-[3-(o-Chlor-p-trifluormethyl-phenoxy)-benzoyl]-S,S-dimethyl-sulfoximin kann gemäss dem nachfolgend beschriebenen fünfstufigen Verfahren hergestellt werden:

a) Zu einer Lösung von 46,57 g Dimethylsulfoximin in 900 ml absolutem Tetrahydrofuran wird während 10 Minuten unter Rühren eine Lösung von 56,11 g Kalium-tert.butylat in 400 ml absolutem Tetrahydrofuran tropfenweise zugegeben, wobei die Temperatur des Reaktionsgemisches auf 35°C ansteigt. Nach Beendigung der Zugabe wird das Gemisch 1 Stunde nachgerührt. Auf diese Weise wird das Dimethylsulfoximin-Kaliumsalz in Suspension gebildet.

b) Zur Dimethylsulfoximin-Kaliumsalz-Suspension wird bei -35°C während 20 Minuten unter Rühren eine Lösung von 99,3 g m-Acetoxybenzoylchlorid in 150 ml absolutem Tetrahydrofuran zugetropft, und das Reaktionsgemisch wird dann 30 Minuten bei -35°C nachgerührt. Es wird auf diese Weise das N-(m-Acetoxybenzoyl)-S,S-dimethyl-sulfoximin in Suspension hergestellt.

c) Man entfernt das Kühlbad, versetzt die gelbe Suspension mit einer Lösung von 40,0 g Natriumhydroxid in 200 ml Wasser und rührt 1,5 Stunden bei Raumtemperatur nach. Danach wird das Zweiphasensystem dreimal mit jeweils 150 ml Aethylacetat ausgeschüttelt, die organische Phase abgetrennt und diese mit 150 ml 2n Natriumhydroxid ausgeschüttelt. Die vereinigten wässrigen Phasen werden mit 32%iger Salzsäure auf ca. pH 2 angesäuert, mit Natriumchlorid gesättigt und anschliessend viermal mit jeweils 700 ml Aethylacetat ausgeschüttelt.

Die vereinigten organischen Extrakte werden über wasserfreiem Natriumsulfat getrocknet und unter vermindertem Druck zur Trockene eingedampft. Der resultierende feste Rückstand wird dann in 1 l Aethylacetat bei 80°C gelöst, die Lösung durch Erhitzen auf ca. 0,5 l eingeengt und der Rückstand auf Raumtemperatur abgekühlt. Nach Abnutschen der resultierenden Kristalle werden diese mit 200 ml n-Hexan nachgewaschen und getrocknet. Man erhält 91,91 g (86,2% der theoretischen Ausbeute) N-(m-Hydroxybenzoyl)-S,S-dimethyl-sulfoximin, Smp. 148-150°C.

d) 21,81 g Natriumhydrid (55%ige Dispersion in Oel) werden in 1,6 l absolutes Dimethylsulfoxid vorgelegt, und anschliessend wird eine Lösung von 106,63 g N-(m-Hydroxy-benzoyl)-S,S-dimethyl-sulfoximin in 550 ml absolutem Dimethylsulfoxid während 15 Minuten unter Rühren zugetropft. Das Natriumsalz des N-(m-Hydroxybenzoyl)-S,S-dimethyl-sulfoximins wird auf diese Weise in situ hergestellt.

e) Man fügt zum Gemisch von d) 0,15 g Kupferpulver (elektrolytisch hergestellt) zu und erhitzt das Gemisch auf 150°C. Anschliessend wird unter gutem Rühren eine Lösung von 107,51 g 1,2-Dichlor-4-trifluormethylbenzol in 300 ml Dimethylsulfoxid während 30 Minuten zugetropft, und das Gemisch wird 45 Minuten bei 150°C und pH 8-9 nachgerührt. Danach wird das Lösungsmittel unter vermindertem Druck weitgehend abdestilliert und der Rückstand in 1 l Methylenchlorid aufgenommen, und die unlöslichen Anteile werden durch Filtration entfernt. Das Filtrat wird der Reihe nach zweimal mit jeweils 250 ml Wasser, mit 600 ml 0,5n Natriumhydroxid und zweimal mit jeweils 250 ml Wasser gewaschen, über wasserfreiem Natriumsulfat getrocknet und zur Trockene eingedampft. Anschliessend wird der ölige Rückstand in 600 ml Diäthyläther gelöst und die Lösung mit 500 ml n-Hexan versetzt und geimpft. Man nutscht die resultierenden Kristalle ab, wäscht sie zweimal mit jeweils 100 ml Diäthyläther und trocknet sie, wobei 99,63 g Produkt erhalten werden. Durch Eindampfen der Mutterlauge zur Trockene und Reinigen des Rückstandes (54,0 g) mit Aethylacetat/n-Hexan (2:1) an einer Säule aus 1,4 kg Kieselgel erhält man weitere 32,13 g Kristalle. Man reinigt das kristalline Produkt (insgesamt 131,76 g) weiter, indem man die Kristalle in 1,4 l Methylenchlorid löst, behandelt die Lösung mit etwas Kohle und engt sie weitgehend ein. Dann wird der Rückstand in 250 ml Diäthyläther gelöst und die Lösung mit 150 ml n-Hexan versetzt und geimpft. Schliesslich werden die resultierenden Kristalle abgenutscht, zweimal mit jeweils 100 ml Diäthyläther gewaschen und getrocknet. Man erhält 109,1 g (55,7% der theoretischen Ausbeute) von N-[3-(o-Chlor-p-trifluormethyl-phenoxy)-benzoyl]-S,S-dimethyl-sulfoximin, Smp. 110-113°C.

## II. Formulierungsbeispiele:

### Beispiel 10

Emulgierbares Konzentrat

| | |
|---|---|
| Wirkstoff der Formel I | 250 g |
| Ricinusöl-(20)-äthoxylat | 50 g |
| Dodecylbenzolsulfonsäure-Calciumsalz | 25 g |
| Gemisch aus o-, m- und p-Xylolen | 150 g |
| N-Methyl-2-pyrrolidon | auf 1000 ml |

Der Wirkstoff, das Ricinusöl-(20)-äthoxylat (nichtionogenaktiver Emulgator) und das Dodecylbenzolsulfonsäure-Calciumsalz (anionaktiver Emulgator) werden im Xylol-Gemisch (Hilfslösungsmittel) und in einem Teil des N-Methyl-2-pyrrolidons (Hauptlösungsmittel) gelöst, und das Ganze wird anschliessend mit einer weiteren Menge N-Methyl-2-pyrrolidon auf das Volumen 1000 ml gebracht.

Das so erhaltene emulgierbare Konzentrat kann mit Wasser verdünnt werden, um eine stundenlang stabile Emulsion herzustellen. Eine solche Emulsion eignet sich als gebrauchsfertige Spritzbrühe.

### Beispiel 11

Zur Herstellung eines emulgierbaren Konzentrates (1,2,3,4 oder 5) werden die nachstehend aufgeführten Bestandteile miteinander vermischt:

Emulgierbares Konzentrat 1

| | |
|---|---|
| Wirkstoff der Formel I | 250 g |
| N-Methyl-2-pyrrolidon | 300 g |
| Emulgator A[1] | 100 g |
| Emulgator B[2] | 25 g |
| Lösungsmittelgemisch aus Alkylbenzolen | auf 1000 ml |

[1]Emulgator bestehend aus 60 Teilen eines Aethylenoxid-Propylenoxid-Blockpolymerisates, 20 Teilen Dodecylbenzolsulfonsäure-Calciumsalz und 20 Teilen Isobutanol/ $C_{10}$-Alkylbenzole-Gemisch.

[2]Emulgator bestehend aus 70 Teilen Dodecylbenzolsulfonsäure-Calciumsalz und 30 Teilen Isobutanol/$C_{10}$-Alkylbenzole-Gemisch.

Emulgierbares Konzentrat 2

| | |
|---|---|
| Wirkstoff der Formel I | 250 g |
| Dimethylformamid | 300 g |
| Fettalkohol-(10)-äthoxylat | 75 g |
| Dodecylbenzolsulfonsäure-Calciumsalz | 25 g |
| Lösungsmittelgemisch aus Alkylbenzolen | auf 1000 ml |

Emulgierbares Konzentrat 3

| | |
|---|---|
| Wirkstoff der Formel I | 500 g |
| Ricinusöl-(30)-äthoxylat | 50 g |
| Dodecylbenzolsulfonsäure-Calciumsalz | 25 g |
| Cyclohexanon | auf 1000 ml |

Emulgierbares Konzentrat 4

| | |
|---|---|
| Wirkstoff der Formel I | 750 g |
| Alkylbenzol-(8)-äthoxylat | 50 g |
| Alkylbenzolsulfonsäure-Ammoniumsalz | 25 g |
| Lösungsmittelgemisch aus Xylolen | auf 1000 ml |

Emulgierbares Konzentrat 5

| | |
|---|---|
| Wirkstoff der Formel I | 250 g |
| Ricinusöl-(20)-äthoxylat | 50 g |
| Dodecylbenzolsulfonsäure-Calciumsalz | 25 g |
| Cyclohexanon | 200 g |
| Lösungsmittelgemisch aus Alkylbenzolen | auf 1000 ml |

Das so erhaltene Konzentrat (1,2,3,4 bzw. 5) emulgiert spontan in Wasser, und die sich ergebende Emulsion eignet sich als gebrauchsfertige Spritzbrühe.

Beispiel 12

Zur Herstellung eines Spritzpulvers (1 oder 2) werden die nachstehend aufgeführten Bestandteile miteinander vermischt und hierauf feingemahlen:

Spritzpulver 1

| | Gewichtsprozent |
|---|---|
| Wirkstoff der Formel I | 50 |
| Hydratisierte Kieselsäure | 5 |
| Natrium-laurylsulfat | 1 |
| Natrium-lignosulfonat | 2 |
| Kaolin | 42 |

Spritzpulver 2

| | Gewichtsprozent |
|---|---|
| Wirkstoff der Formel I | 25 |
| Hydratisierte Kieselsäure | 30 |
| Alkylphenol-(12)-äthoxylat | 4 |
| Polycarbonsäure-Natriumsalz | 4 |
| Kaolin | 37 |

Das so erhaltene Pulver (1 bzw. 2) kann mit Wasser benetzt werden. Die entstandene Suspension eignet sich als gebrauchsfertige Spritzbrühe.

## Beispiel 13

Zur Herstellung eines Stäubemittels werden die nachstehend aufgeführten Bestandteile miteinander vermischt und hierauf feingemahlen:

| | Gewichtsprozent |
|---|---|
| Wirkstoff der Formel I | 10 |
| Hydratisierte Kieselsäure | 7,5 |
| Talkum | 82,5 |

## Beispiel 14

Zur Herstellung eines Granulats werden die nachstehend aufgeführten Bestandteile miteinander vermischt:

| | Gewichtsprozent |
|---|---|
| Wirkstoff der Formel I | 5 |
| Dipropylenglykol | 5 |
| Bimssteingranulat | 90 |

## Patentansprüche

1. Verbindungen der allgemeinen Formel

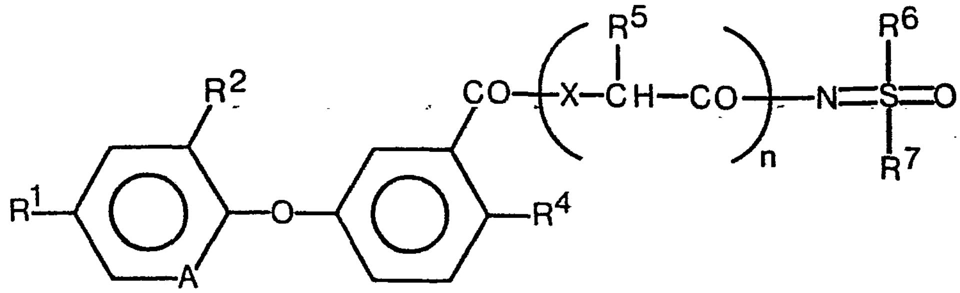

I

worin A N oder $CR^3$,
$R^1$ Halogen oder Trifluormethyl,
$R^2$ und $R^3$, unabhängig voneinander, Wasserstoff oder Halogen,
$R^4$ Halogen, Nitro oder Cyano,
$R^5$ Wasserstoff oder Methyl,
$R^6$ $C_{1-6}$-Alkyl und $R^7$ $C_{1-6}$-Alkyl oder gegebenenfalls mit Halogen, $C_{1-4}$-Alkyl, $C_{1-4}$-Alkoxy und/oder Nitro substituiertes Aryl, oder $R^6$ und $R^7$ zusammen Tetra-, Penta- oder Hexamethylen,
n 0 oder 1,
X Sauerstoff oder $-NR^8-$
und $R^8$ Wasserstoff oder $C_{1-6}$-Alkyl bedeuten.

2. Verbindungen nach Anspruch 1, worin $R^6$ $C_{1-6}$-Alkyl und $R^7$ $C_{1-6}$-Alkyl oder gegebenenfalls mit Halogen, $C_{1-4}$-Alkyl, $C_{1-4}$-Alkoxy und/oder Nitro substituiertes Aryl bedeuten.

3. Verbindungen nach Anspruch 1 oder 2, worin A $CR^3$, $R^1$ Trifluormethyl, $R^2$ Chlor und $R^3$ Wasserstoff bedeuten.

4. Verbindungen nach einem der Ansprüche 1 bis 3, worin $R^4$ Nitro bedeutet.

5. Verbindungen nach einem der Ansprüche 1 bis 4, worin X Sauerstoff bedeutet.

6. N-[5-(o-Chlor-p-trifluormethyl-phenoxy)-2-nitrobenzoyl]-S,S-dimethyl-sulfoximin.

7. Eine Verbindung nach Anspruch 1, ausgewählt aus:

N-[5-(o-Chlor-p-trifluormethyl-phenoxy)-2-nitrobenzoyl]-S-äthyl-S-methyl- sulfoximin,

N-[5-(o-Chlor-p-trifluormethyl-phenoxy)-2-nitrobenzoyl]-S,S-diäthyl-sulfoximin,

N-[5-(o-Chlor-p-trifluormethyl-phenoxy)-2-nitrobenzoyl]-S-methyl-S-phenyl-sulfoximin,

N-[5-(o-Chlor-p-trifluormethyl-phenoxy)-2-nitrobenzoyl]-S-(n-hexyl)-S-methyl-sulfoximin,

N-{2-[5-(o-Chlor-p-trifluormethyl-phenoxy)-2-nitrobenzoyloxy]-propionyl}-S-methyl-S-phenyl-sulfoximin und

N-{2-[5-(o-Chlor-p-trifluormethyl-phenoxy)-2-nitrobenzoyloxy]-propionyl}-S-äthyl-S-methyl-sulfoximin.

8. Eine Verbindung nach Anspruch 1, ausgewählt aus:

N-[5-(o-Chlor-p-trifluormethyl-phenoxy)-2-nitrobenzoyl]-S-methyl-S-(p-chlorphenyl)-sulfoximin,

N-[5-(o-Chlor-p-trifluormethyl-phenoxy)-2-nitrobenzoyl]-S-methyl-S-(p-tolyl)-sulfoximin,

N-[5-(o-Chlor-p-trifluormethyl-phenoxy)-2-nitrobenzoyl]-S-methyl-S-(o-chlorphenyl)-sulfoximin,

N-[5-(5-Trifluormethyl-2-pyridyloxy)-2-nitrobenzoyl]-S,S-dimethyl-sulfoximin,

N-[5-(3,5-Dichlor-2-pyridyloxy)-2-nitrobenzoyl]-S,S-dimethyl-sulfoximin,

N-[5-(3-Chlor-5-trifluormethyl-2-pyridyloxy)-2-nitrobenzoyl]-S,S-dimethyl-sulfoximin,

N-[2-Chlor-5-(o-chlor-p-trifluormethyl-phenoxy)-benzoyl]-S,S-dimethyl-sulfoximin,

N-[5-(o-Chlor-p-trifluormethyl-phenoxy)-2-jodbenzoyl]-S,S-dimethyl-sulfoximin,

N-[5-(o-Chlor-p-trifluormethyl-phenoxy)-2-nitro-benzoyl]-S,S-tetramethylen-sulfoximin,

N-[5-(o-Chlor-p-trifluormethyl-phenoxy)-2-nitro-benzoyl]-S-methyl-S-(p-nitrophenyl)-sulfoximin,

N-[5-(o-Chlor-p-trifluormethyl-phenoxy)-2-nitro-benzoyl]-S-methyl-S-(p-methoxyphenyl)-sulfoximin,

N-{2-[5-(o-Chlor-p-trifluormethyl-phenoxy)-2-nitro-benzoyloxy]-propionyl}-S-methyl-S-(p-chlorphenyl)-sulfoximin,

N-{2-[5-(o-Chlor-p-trifluormethyl-phenoxy)-2-nitro-benzoyloxy]-propionyl}-S-methyl-S-(p-tolyl)-sulfoximin,

N-{2-[5-(o-Chlor-p-trifluormethyl-phenoxy)-2-nitro-benzoyloxy]-propionyl}-S,S-tetramethylen-sulfoximin,

N-{[5-(o-Chlor-p-trifluormethyl-phenoxy)-2-nitro-benzoyloxy]acetyl}-S,S-dimethyl-sulfoximin,

N-{2-[5-(o-Chlor-p-trifluormethyl-phenoxy)-2-nitro-benzoyloxy]-propionyl}-S,S-dimethyl-sulfoximin,

N-{2-[5-(o-Chlor-p-trifluormethyl-phenoxy)-2-nitro-benzoyloxy]-propionyl}-S-methyl-S-(p-methoxyphenyl)-sulfoximin und

N-{2-[5-(o-Chlor-p-trifluormethyl-phenoxy)-2-nitro-benzoyloxy]-propionyl}-S-methyl-S-(p-nitrophenyl)-sulfoximin.

9. Eine Verbindung nach Anspruch 1 als Unkrautbekämpfungsmittel.

10. Unkrautbekämpfungsmittel, dadurch gekennzeichnet, dass es eine wirksame Menge mindestens einer Verbindung der allgemeinen Formel

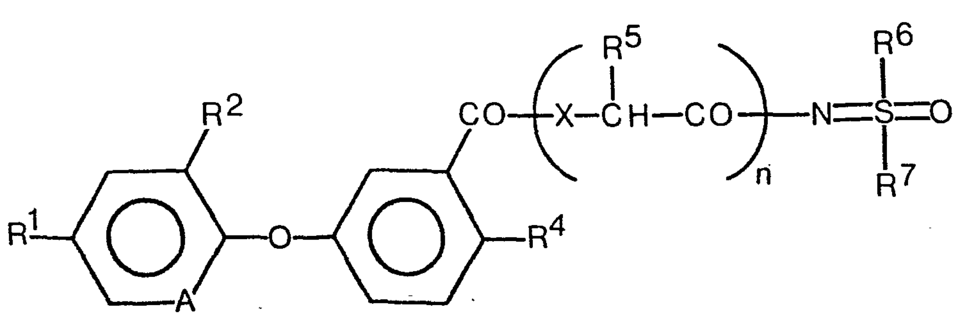

I

worin A N oder $CR^3$,

$R^1$ Halogen oder Trifluormethyl,

$R^2$ und $R^3$, unabhängig voneinander, Wasserstoff oder Halogen,

$R^4$ Halogen, Nitro oder Cyano,

$R^5$ Wasserstoff oder Methyl,

$R^6$ $C_{1-6}$-Alkyl und $R^7$ $C_{1-6}$-Alkyl oder gegebenenfalls mit Halogen, $C_{1-4}$-Alkyl, $C_{1-4}$-Alkoxy und/oder Nitro substituiertes Aryl, oder $R^6$ und $R^7$ zusammen Tetra-, Penta- oder Hexamethylen,

n 0 oder 1,

X Sauerstoff oder $-NR^8-$

und $R^8$ Wasserstoff oder $C_{1-6}$-Alkyl bedeuten,

sowie Formulierungshilfsstoffe enthält.

11. Unkrautbekämpfungsmittel nach Anspruch 10, dadurch gekennzeichnet, dass es eine wirksame Menge N-[5-(o-Chlor-p-trifluormethyl-phenoxy)-2-nitrobenzoyl]-S,S-dimethylsulfoximin sowie Formulierungshilfsstoffe enthält.

12. Verfahren zur Herstellung von Verbindungen der allgemeinen Formel

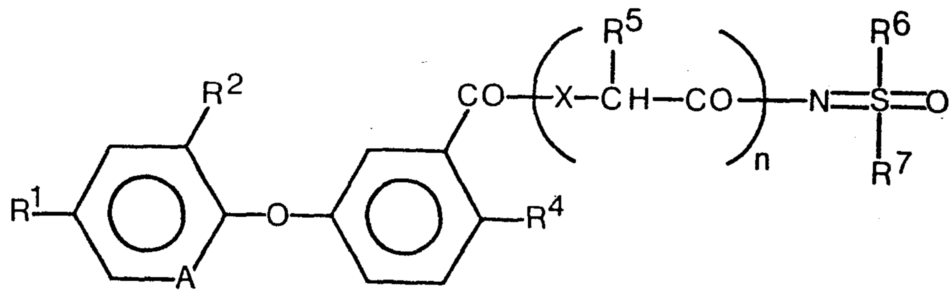

I

worin A N oder $CR^3$,

$R^1$ Halogen oder Trifluormethyl,

$R^2$ und $R^3$, unabhängig voneinander, Wasserstoff oder Halogen,

$R^4$ Halogen, Nitro oder Cyano,

$R^5$ Wasserstoff oder Methyl,

$R^6$ $C_{1-6}$-Alkyl und $R^7$ $C_{1-6}$Alkyl oder gegebenenfalls mit Halogen, $C_{1-4}$-Alkyl, $C_{1-4}$-Alkoxy und/oder Nitro substituiertes Aryl, oder

$R^6$ und $R^7$ zusammen Tetra-, Penta- oder Hexamethylen,

n 0 oder 1,

X Sauerstoff oder $-NR^8-$

und $R^8$ Wasserstoff oder $C_{1-6}$-Alkyl bedeuten,

dadurch gekennzeichnet, dass man

a) eine Säure der allgemeinen Formel

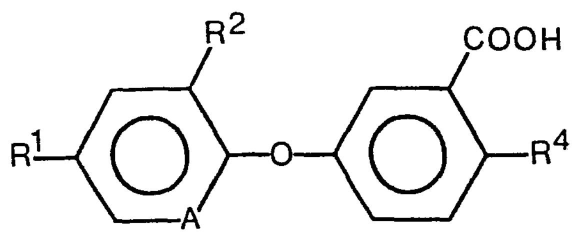

II

worin A, $R^1$, $R^2$ und $R^4$ die oben angegebenen Bedeutungen besitzen,

in Form eines reaktionsfähigen Derivats davon mit einem Sulfoximin der allgemeinen Formel

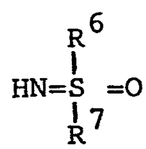

III

worin $R^6$ und $R^7$ die oben angegebenen Bedeutungen besitzen,

oder mit einem Alkalimetallsalz davon umsetzt,

b) ein Salz oder reaktionsfähiges Derivat einer Säure der oben angegebenen allgemeinen Formel II mit einer Verbindung der allgemeinen Formel

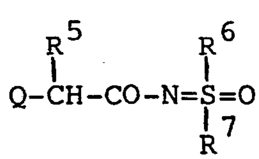

IV

worin $R^5$, $R^6$ und $R^7$ die oben angegebenen Bedeutungen besitzen und Q eine Abgangsgruppe bzw. Hydroxy

bedeutet,

umsetzt,

c) eine Verbindung der allgemeinen Formel

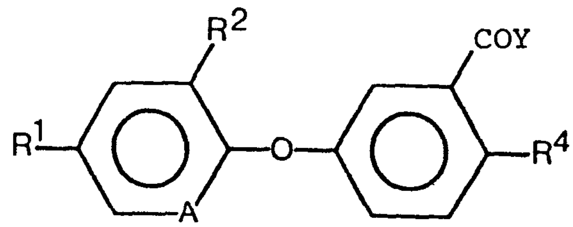

V

worin A, $R^1$, $R^2$ und $R^4$ die oben angegebenen Bedeutungen besitzen,

Y -NHR$^8$ oder Halogen bedeutet

und $R^8$ die oben angegebene Bedeutung besitzt,

mit einer Verbindung der allgemeinen Formel

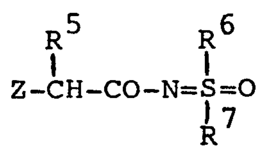

VI

worin $R^5$, $R^6$ und $R^7$ die oben angegebenen Bedeutungen besitzen,

und Z im Falle von Y = -NHR$^8$ für eine Abgangsgruppe, und im Falle von Y = Halogen für -NHR$^8$ steht,

umsetzt,

d) ein Phenol der allgemeinen Formel

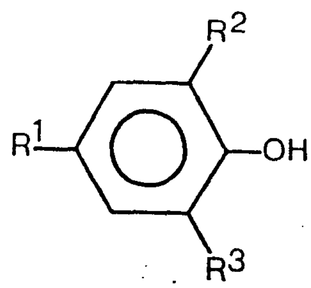

VII

worin $R^1$, $R^2$ und $R^3$ die oben angegebenen Bedeutungen besitzen,

oder ein Alkalimetallsalz davon mit einem Halogenid der allgemeinen Formel

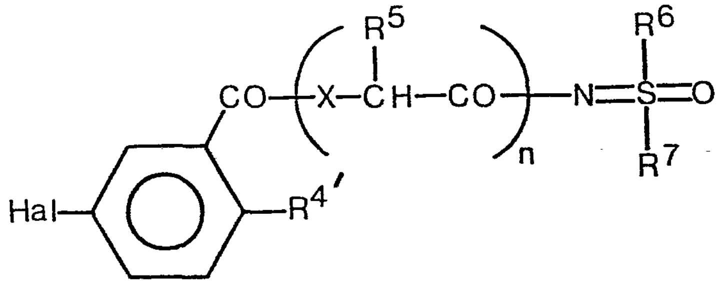

VIII

worin $R^5$, $R^6$, $R^7$, n und X die oben angegebenen Bedeutungen besitzen,
$R^{4'}$ Nitro oder Cyano bedeutet
und Hal Halogen bedeutet,
umsetzt,

e) ein Hydroxybenzoesäurederivat der allgemeinen Formel

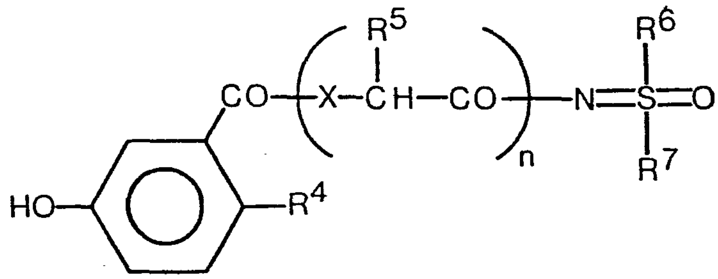

IX

worin $R^4$, $R^5$, $R^6$, $R^7$, n und X die oben angegebenen Bedeutungen besitzen,
oder ein Alkalimetallsalz davon mit einem Halogenid der allgemeinen Formel

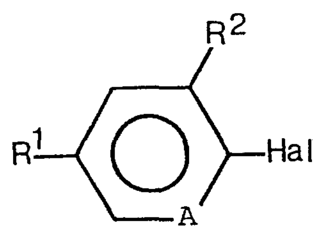

X

worin A, $R^1$ und $R^2$ die oben angegebenen Bedeutungen besitzen und Hal Halogen bedeutet, wobei, falls A $CR^3$ und $R^2$ und/oder $R^3$ Halogen darstellen, $R^1$ Trifluormethyl bedeutet,

umsetzt, oder

f) eine Verbindung der allgemeinen Formel

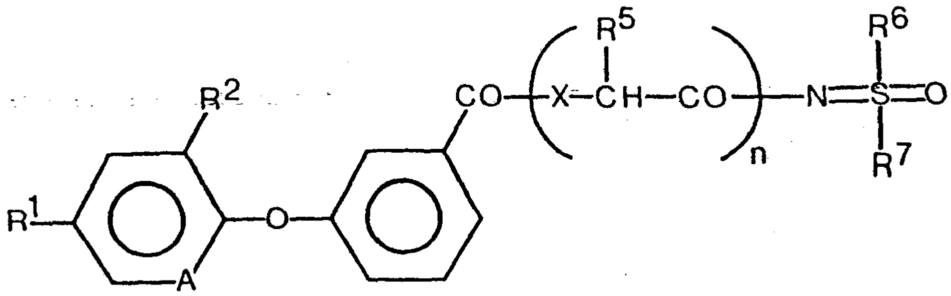

XI

worin A, $R^1$, $R^2$, $R^5$, $R^6$, $R^7$, n und X die oben angegebenen Bedeutungen besitzen,

nitriert.

13. Verfahren zur Bekämpfung von Unkräutern, dadurch gekennzeichnet, dass man das gegen Unkräuter zu schützende Gut und/oder die Unkräuter mit einer wirksamen Menge einer der in den Ansprüchen 1 bis 8 genannten Verbindungen bzw. eines in Anspruch 10 oder 11 genannten Mittels behandelt.

14. Verwendung einer der in den Ansprüchen 1 bis 8 genannten Verbindungen bzw. eines in Anspruch 10 oder 11 genannten Mittels zur Bekämpfung von Unkräutern.

15. N-(5-Fluor-2-nitrobenzoyl)-S,S-dimethyl-sulfoximin.

16. N-(5-Hydroxy-2-nitrobenzoyl)-S,S-dimethyl-sulfoximin.

17. Eine Verbindung der allgemeinen Formel

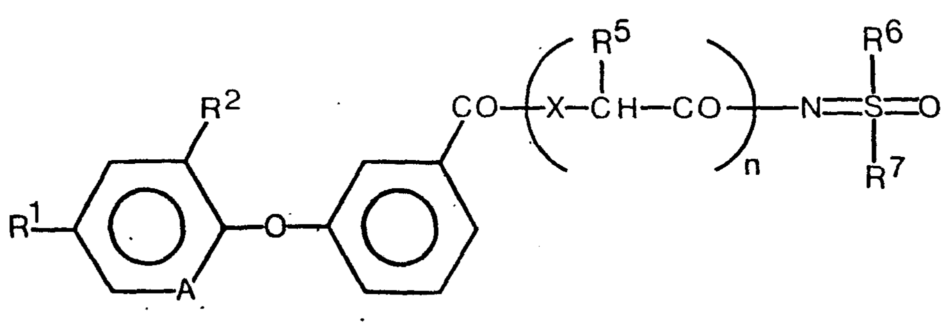

XI

worin A N oder $CR^3$,
$R^1$ Halogen oder Trifluormethyl,
$R^2$ und $R^3$, unabhängig voneinander, Wasserstoff oder Halogen,
$R^5$ Wasserstoff oder Methyl,
$R^6$ $C_{1-6}$-Alkyl und $R^7$ $C_{1-6}$-Alkyl oder gegebenenfalls mit Halogen, $C_{1-4}$-Alkyl, $C_{1-4}$-Alkoxy und/oder Nitro substituiertes Aryl, oder $R^6$ und $R^7$ zusammen Tetra-, Penta- oder Hexamethylen,
n 0 oder 1,
X Sauerstoff oder $-NR^8-$
und $R^8$ Wasserstoff oder $C_{1-6}$-Alkyl bedeuten.

18. N-[3-(o-Chlor-p-trifluormethyl-phenoxy)-benzoyl]-S,S-dimethyl-sulfoximin.

***